# EUROPÄISCHE PATENTSCHRIFT

(11) **EP 2 630 176 B1**
(45) Veröffentlichungstag und Bekanntmachung des Hinweises auf die Patenterteilung: **01.07.2015**
(21) Anmeldenummer: 11770807.3
(22) Anmeldetag: 20.10.2011
(51) Int. Cl.: C08G 18/22

(54) **POLYURETHANVERDICKER**
POLYURETHANE THICKENER
ÉPAISSISSANTS POLYURÉTHANES

(30) Priorität: 22.10.2010 EP 10188535
(43) Veröffentlichungstag der Anmeldung: 28.08.2013
(73) Patentinhaber: BASF SE, 67056 Ludwigshafen (DE)
(72) Erfinder: TÜRK, Holger, 68161 Mannheim (DE); WENDEL, Volker, 64342 Seeheim-Jugenheim (DE)
(74) Vertreter: Blumberg vel Spalve, Jutta
(86) Internationale Anmeldenummer: PCT/EP2011/068330
(87) Internationale Veröffentlichungsnummer: WO 2012/052508

(56) Entgegenhaltungen:
- EP-A1- 0 639 595
- EP-A1- 0 812 608
- WO-A2-2009/135857
- DE-A1-102004 031 786
- US-A1- 2009 318 595

## Beschreibung

Die vorliegende Erfindung betrifft ein Verfahren zur Herstellung von Polyurethanen, die mindestens drei hydrophile Abschnitte, mindestens vier hydrophobe Abschnitte, gegebenenfalls Allophanat-Segmente und/oder Isocyanurat-Segmente enthalten und die in Gegenwart von (Erd)alkalimetallcarboxylaten hergestellt werden.

Weiterhin betrifft die vorliegende Erfindung die auf diese Weise erhältlichen Polyurethane an sich, deren Verwendung als Verdicker für wässrige Zubereitungen sowie Zubereitungen enthaltend derartige Polyurethane.

Seit langem werden Polyurethane in zahlreichen Anwendungsgebieten für unterschiedlichste Zwecke eingesetzt. Entsprechend der Wahl der Ausgangsstoffe und des stöchiometrischen Verhältnisses der Ausgangsstoffe erhält man Polyurethane mit sehr unterschiedlichen physikalisch-chemischen Eigenschaften.

Verdickungsmittel werden in großem Umfang zur Erhöhung der Viskosität von wässrigen Zubereitungen eingesetzt, beispielsweise auf den Gebieten Kosmetik, Human- und Tierernährung, Pharmazie und für Waschmittel, Farben und Lacke. Unter anderem sind auch Polyurethane als Verdickungsmittel bekannt.

Beispielsweise werden Polyurethanlösungen oder -dispersionen in wasserverdünnbarer wässriger oder überwiegend wässriger Phase vom Fachmann als HEUR-Verdicker bezeichnet ("hydrophobically modified ethylene oxide urethane copolymer"), und bereits seit längerem in den unterschiedlichsten Anwendungsfeldern, beispielsweise zur Verdickung wasserbasierter Dispersionsfarben, eingesetzt.

Als Wirkprinzip für die verdickende Wirkung der HEUR-Verdicker in vorgenanntem Beispiel wird angenommen, dass die Polyethylenglykolsegmente die Wasserverträglichkeit sicherstellen und die Hydrophobsegmente über eine Assoziation untereinander sowie mit dispergierten Bindemittelteilchen der zu verdickenden Dispersionsfarbe in dieser einen viskositätsgebenden dreidimensionalen Molekülverbund aufbauen.

Auch im Bereich der kosmetischen Zubereitungen werden Verdicker eingesetzt. Von Verdickern für kosmetische Zubereitungen wird eine ausreichende Verdickungswirkung auch in stark salzhaltigen Zubereitungen erwartet. Weiterhin sollen solche Verdicker kosmetische Zubereitungen mit guter Textur und angenehmen Gefühl auf der Haut erzeugen. Die Verträglichkeit mit zahlreichen anderen Hilfsstoffen, insbesondere mit Salzen und Tensiden und auch die Einarbeitbarkeit des Verdickers selbst sowie der weiteren Hilfsstoffe sollte gegeben sein.

Außerdem müssen die verdickten Zubereitungen auch bei Langzeitlagerung, Temperatur- und pH-Veränderungen eine im Wesentlichen gleichbleibende Rheologie und physikalische und chemische Qualität aufweisen. Letztlich sollen diese Verdicker noch kostengünstig und ohne eine merkliche Umweltbelastung herzustellen sein.

US 4079028 und US 4155892 offenbaren unter anderem lineare Polyurethan-Verdicker. Die Herstellung der dort genannten Polyurethane wird durch den in der Polyurethanchemie üblichen Katalysator Dibutylzinndilaurat (DBTL) katalysiert.

EP 1584331 und EP 1013264 beschreiben Polyurethan-Verdicker für kosmetische Zubereitungen. Diese werden in einem Ein-Schritt-Verfahren durch Reaktion in Substanz aus Polyol, Polyisocyanat und Fettalkohol, der gewünschtenfalls ethoxyliert sein kann, und ohne Verwendung eines Katalysators hergestellt.

WO 2006/002813 beschreibt Polyurethan-Verdicker für verschiedene Anwendungen in wässrigen Medien. Diese Verdicker werden aus hydrophilen Polyolen mit mindestens zwei Hydroxygruppen, ein oder mehr hydrophoben Verbindungen, z.B. langkettigen Alkoholen, und mindestens difunktionalen Isocyanaten hergestellt. Dabei wird ein Überschuss an NCO-Gruppen eingesetzt. Als Katalysator wird 1,8-Diazabicyclo-[5-4-0]undec-7-en (DABCO) verwendet.

WO 02/88212 beschreibt Polyurethane aus ethoxylierten langkettigen Alkoholen und cyclischen Diisocyanat-Oligomeren, beispielsweise Isocyanuraten, als Ausgangsmaterialien. Die beschriebenen Polyurethane werden ohne Verwendung eines Katalysators hergestellt.

EP 725097 beschreibt Polyurethan-Verdicker, bei deren Herstellung Polyether, erzeugt durch Alkoxylierung von Alkoholen oder Alkylphenolen, mit Polyisocyanaten unter Katalyse durch DBTL, Diazabicyclooctan, oder Zinndioctoat umgesetzt werden, wobei das Verhältnis von NCO zu OH-Äquivalenten im Bereich von 0,9:1 bis 1,2:1 liegt. Diese Verdicker werden für den Einsatz im Bereich niedriger Scherkräfte z.B. für den Verlauf von wässrigen Dispersionsfarben vorgeschlagen.

EP 1241198, EP 1241199 EP 0 639 595 und EP 1241200 beschreiben die Herstellung von Polyurethan-Verdickern unter DBTL-Katalyse und Verwendung von Polyetherpolyolen bzw. urethangruppenhaltige Polyetherpolyolen mit Funktionalitäten >2 (z.B. ethoxylierte Zucker, Glycerin etc.).

EP 761780 und EP 1111014 beschreiben Polyurethan-Verdicker aufgebaut aus Polyethylenglykol, Diisocyanat und verzweigten, bevorzugt Langketten-verzweigten Alkylgruppen als hydrophobe Komponente. Die Herstellung erfolgt in der Schmelze ohne Verwendung eines Katalysators.

WO 2009/135856 und WO 2009/135857 beschreiben in Wasser dispergierbare Polyurethane mit einem im Wesentlichen linearen Rückgrat aufgebaut aus alternierenden hydrophilen und hydrophoben Abschnitten und deren Verwendungen. Die Polyurethanbildung wird durch Titan- oder Zinkverbindungen katalysiert.

Eine Aufgabe der vorliegenden Erfindung war es, neue Verdicker für Wasser enthaltende Zubereitungen, insbesondere kosmetische Zubereitungen, bereitzustellen. Die neuen Verdicker sollten eine möglichst gute Verdickungswirkung aufweisen. Darüber hinaus sollte die Verdickungswirkung der neuen Verdicker durch die Gegenwart von Salzen in den wässrigen Zubereitungen wenigstens nicht vermindert werden.

Ein weiteres Ziel war die Bereitstellung von Polyurethanverdickern mit den beschriebenen Eigenschaften, die zusätzlich zinnfrei sind, da dies für kosmetische Anwendungen erwünscht ist.

Die vorstehenden Aufgaben werden gelöst durch ein Verfahren zur Herstellung von Polyurethanen umfassend
I) mindestens zwei hydrophile Abschnitte S,
II) mindestens einen von S verschiedenen hydrophilen Abschnitt P,
III) mindestens zwei endständige hydrophobe Abschnitte T,
IV) mindestens zwei von T verschiedene hydrophobe Abschnitte D,
   wobei
   a) sich an jeden Abschnitt T ein Abschnitt S unmittelbar anschließt,
   b) sich an jeden Abschnitt S auf mindestens einer Seite mindestens ein Abschnitt D anschließt,
   c) sich an jeden Abschnitt P mindestens zwei Abschnitte D anschließen,
dadurch gekennzeichnet,
dass die Herstellung in Gegenwart mindestens eines Carbonsäuresalzes eines Metalls ausgewählt aus der Gruppe bestehend aus Alkalimetallen, Erdalkalimetallen und deren Mischungen erfolgt.

Bei den durch das erfindungsgemäße Verfahren erhältlichen Polyurethanen handelt es sich um Gemische, welche die beschriebenen Polyurethanstrukturen mindestens zum Teil enthalten. Die nach dem erfindungsgemäßen Verfahren erhältlichen Polyurethane sind bevorzugt in Wasser dispergierbar. Erfindungsgemäß umfasst dies, dass sie sich auch in Wasser emulgieren lassen oder in Wasser vollständig oder teilweise löslich sind.

Die nach dem erfindungsgemäßen Verfahren erhältlichen Polyurethane (im Folgenden auch als "erfindungsgemäße Polyurethane" bezeichnet) sind bevorzugt wenigstens teilweise verzweigt. "Wenigstens teilweise verzweigt" bedeutet, dass wenigstens ein Teil der Polymermoleküle nicht linear ist, sondern Verzweigungsstellen aufweist.

Solche Verzweigungen können sowohl in den hydrophoben als auch den hydrophilen Abschnitten enthalten sein.

In einer Ausführungsform der Erfindung ist wenigstens ein Teil der endständigen hydrophoben Abschnitte T verzweigt.

In einer Ausführungsform der Erfindung ist wenigstens ein Teil der hydrophoben Abschnitte D verzweigt.

Ein Vorteil der erfindungsgemäß erhältlichen Polyurethane ist, dass durch die Verwendung von (Erd)alkalycarboxylaten Verzweigungen der Polyisocyanate in Form von Isocyanurat- oder Allophanatstrukturen in-situ erzeugt werden können und somit nicht auf Polyisocyanate mit bereits vorgefertigten Isocyanurat- oder Allophanatstrukturen als Ausgangsverbindungen zurückgegriffen werden muss. Zum einen werden die Ausgangsmaterialien dadurch preislich günstiger und zum anderen kann die gewünschte Menge an derartigen Verzweigungsstellen durch die Menge des Katalysators auf das gewünschte Maß eingestellt werden. Weiterhin führt das erfindungsgemäße Verfahren weniger schnell zu vernetzten Strukturen als die Verwendung von vorgefertigten Isocyanurat- oder Allophanatstrukturen.

Das Rückgrat der erfindungsgemäßen Polyurethane ist aufgebaut aus alternierenden hydrophoben und hydrophilen Abschnitten, wobei die hydrophoben und hydrophilen Abschnitte sich zwar in der Abfolge abwechseln, aber in ihrer Größe, Länge und Natur unterschiedlich sein können. In den erfindungsgemäßen Polyurethanen schließt sich bevorzugt an einen hydrophilen Abschnitt an beiden Seiten ein hydrophober Abschnitt an. Diese hydrophoben Abschnitte können unabhängig voneinander gleich oder verschieden sein. Jeder Abschnitt kann kurzkettig oder ein Oligomerrest oder ein Polymerrest sein. Unter "sich an einen Abschnitt anschließen" wird vorliegend verstanden, dass der Anschluss unmittelbar erfolgt, d.h. dass die beiden betreffenden Abschnitte im Polymermolekül direkt benachbart sind.

### Hydrophile Abschnitte

Hydrophil bezeichnet dabei solche Abschnitte, die eine ausgeprägte Wechselwirkung mit Wasser zeigen. Im Allgemeinen bestehen hydrophile Abschnitte aus Resten von Stoffen, die selbst hydrophil sind.

Dem Fachmann bekannte typische hydrophile Gruppen sind beispielsweise nichtionische Polyether-Reste. Polyether-Reste können homo-Alkylenoxidreste sein oder Mischungen unterschiedlicher Alkylenoxidreste enthalten. Diese unterschiedlichen Alkylenoxidreste können statistisch verteilt in den Polyether-Resten vorhanden sein oder blockförmig vorliegen. Bevorzugte Polyether-Reste sind homo-Ethylenoxidreste. Im Folgenden wird Ethylenoxid auch als EO, Propylenoxid auch als PO bezeichnet.

Nach einer anderen Ausführungsform enthalten die Polyether-Reste Mischungen aus EO-Resten und PO-Resten. Diese können statistisch verteilt in den Polyether-Resten vorhanden sein oder blockförmig vorliegen. In einer bevorzugten Ausführungsform liegen die EO- und PO-Reste blockförmig vor.

Zu einer besonders bevorzugten Ausführungsform gehören Polyether-Reste, die mindestens 50 Gew.-% Ethylenoxidreste aufweisen, beispielsweise Polyether-Reste, die über 50 Gew.-% Ethylenoxidreste und als weitere Alkylenoxidreste Propylenoxidreste aufweisen. Ganz besonders bevorzugt bestehen die Polyether-Reste aus Ethylenoxidresten.

Die Hydrophilie eines Stoffes kann beispielsweise durch eine Trübungsmessung einer wässrigen Lösung bestimmt werden.

Bevorzugte hydrophile Abschnitte sind wasserlöslich. Eine Substanz wird zu den Zwecken dieser Erfindung als in einer flüssigen Phase löslich bezeichnet, wenn mindestens 1 g, bevorzugt mindestens 10 g der Substanz bei 20°C und 1 bar Druck für das menschliche Auge klar, d.h. ohne sichtbare Trübung, in 1 Liter der flüssigen Phase löslich sind. Wasserlöslich sind somit Substanzen, die in einer Menge von mindestens 1 g, bevorzugt mindestens 10 g bei 20°C und 1 bar Druck für das menschliche Auge klar, d.h. ohne sichtbare Trübung, in 1 Liter Wasser, bevorzugt voll entsalztem Wasser, löslich sind.

### Hydrophobe Abschnitte

Durch das erfindungsgemäße Verfahren werden Polyurethanmoleküle erhalten, die wenigstens zwei endständige hydrophobe Abschnitte T und wenigstens zwei hydrophobe Abschnitte D enthalten.

Es werden aber, je nachdem, wie die Reaktion geführt wird, auch Polyurethanmoleküle erhalten, die zwei hydrophobe Abschnitte T und nur einen hydrophoben Abschnitt D enthalten.

Im Allgemeinen bestehen die hydrophoben Abschnitte aus Resten von Substanzen, die nicht oder nur schlecht mit Wasser mischbar sind und bevorzugt gleichzeitig lipophil sind, d.h. in unpolaren Lösungsmitteln wie beispielsweise Fetten und Ölen gut löslich sind.

Typische hydrophobe Abschnitte T sind beispielsweise Kohlenwasserstoffreste, insbesondere langkettige Kohlenwasserstoffreste.

In einer Ausführungsform der Erfindung sind die Kohlenwasserstoffreste unverzweigt. In einer anderen Ausführungsform der Erfindung sind die Kohlenwasserstoffreste verzweigt.

In einer weiteren Ausführungsform der Erfindung enthalten die erfindungsgemäßen Polyurethane sowohl verzweigte als auch unverzweigte Kohlenwasserstoffreste.

Langkettige aliphatische Alkohole, aromatische Alkohole sowie aliphatische Diisocyanate sind Beispiele für hydrophobe Substanzen, deren Reste in den hydrophoben Abschnitten der erfindungsgemäßen Polyurethane enthalten sein können.

Die nach dem erfindungsgemäßen Verfahren hergestellten Polyurethane enthalten mindestens zwei endständige hydrophobe Abschnitte (T), die unabhängig voneinander gleich oder verschieden sein können.

In einer bevorzugten Ausführungsform enthält wenigstens ein Teil der erfindungsgemäßen Polyurethane mehr als zwei endständige hydrophobe Abschnitte (T).

Die endständigen hydrophoben Abschnitte T können verzweigt oder unverzweigt sein. Bevorzugt ist mindestens einer der beiden endständigen hydrophoben Abschnitte T verzweigt.

Bevorzugt enthalten die endständigen hydrophoben Abschnitte T wenigstens einen Alkylrest. Bevorzugt umfasst dieser Alkylrest 4 bis 30 Kohlenstoffatome, besonders 8 bis 30 und ganz besonders bevorzugt 12 bis 30 Kohlenstoffatome.

Bevorzugt liegt die Kettenlänge der Hauptkette der Alkylreste, die in den Abschnitten T enthalten sind, im Bereich von 4 bis 30 Kohlenstoffatomen. Es sind dies beispielsweise Alkylreste von linearen oder verzweigten Alkanen wie beispielsweise Butan, Isobutan, Pentan, Isopentan, Neopentan, Hexan, Heptan, Octan, 2-Ethylhexan, Nonan, Decan, Undecan, Dodecan, Tridecan, Isotridecan, Tetradecan, Pentadecan, Hexadecan, Heptadecan, Octadecan, Nonadecan, Icosan, Henicosan, Docosan, Tricosan, Isotricosan, Tetracosan, Pentacosan, Hexacosan, Heptacosan, Octacosan, Nonacosan, Triacontan, 2-Octyldodecan, 2-Dodecylhexadecan, 2-Tetradecyloctadecan, Monomethyl-verzweigtes Isooctadecan.

Ebenso können die hydrophoben Abschnitte T auch Reste von Cylcoalkanen und -alkenen wie beispielsweise beschrieben in EP 761780 A2, S.4, Z. 56-58, Reste von Alkenen wie beispielsweise beschrieben in EP 761780 A2, S.4, Z. 51-52, oder Alkylaryl-Reste wie beispielsweise beschrieben in EP 761780 A2, S.4, Z. 53-55 umfassen.

Besonders bevorzugt enthalten die Abschnitte T die zuvor beschriebenen Alkylreste mit 8 bis 30 Kohlenstoffatomen, ganz besonders bevorzugt mit 12 bis 30 Kohlenstoffatomen.

Die Abschnitte T bestehen aus oder enthalten bevorzugt die zuvor beschriebenen aliphatischen Reste, können aber auch die zuvor beschriebenen aromatische Reste enthalten oder aus ihnen bestehen.

Bevorzugt ist mindestens ein Abschnitt T ein verzweigter Alkylrest.

In einer bevorzugten Ausführungsform weisen die Seitenketten der verzweigten Alkylreste eine Kettenlänge von höchstens 6, bevorzugt von höchstens 4 Kohlenstoffatomen auf.

In einer Ausführungsform sind die Verzweigungen deutlich kürzer als die Hauptkette. In einer Ausführungsform hat jede Verzweigung der Abschnitte T der erfindungsgemäßen Polyurethane eine Kettenlänge, die höchstens der Hälfte der Kettenlänge der Hauptkette dieses Abschnitts T entspricht. In einer bevorzugten Ausführungsform sind die verzweigten Alkylreste iso- und/oder neo-Alkylreste. In einer bevorzugten Ausführungsform werden als verzweigte Alkylreste Reste von Iso-Alkanen verwendet. Besonders bevorzugt ist ein C₁₃-Alkylrest, insbesondere ein iso-C₁₃-Alkylrest.

In einer anderen Ausführungsform umfassen die Abschnitte T verzweigte Alkylreste, deren Seitenketten eine Kettenlänge von mindestens 4, bevorzugt von mindestens 6 Kohlenstoffatomen aufweisen.

Die Einführung der Abschnitte T in die erfindungsgemäßen Polyurethane kann auf verschiedene Weise erfolgen.

In einer bevorzugten Ausführungsform werden die Abschnitte T gleichzeitig und gemeinsam mit den hydrophilen Abschnitten S durch die Verwendung von alkoxylierten Alkoholen in die Polyurethane eingebracht.

Geeignete Alkohole sind beispielsweise die alkoxylierten
- linearen Alkohole aus natürlichen Quellen oder aus der Ziegler-Aufbaureaktion von Ethylen in Gegenwart von Aluminiumalkyl-Katalysatoren. Beispiele für geeignete Alkohole sind C₆-C₃₀-Alkohole, insbesondere C₁₂-C₃₀-Alkohole. Als besonders bevorzugte Alkohole seien hier genannt: n-Dodecanol, n-Tetradecanol, n- Hexadecanol, n-Octadecanol, n-Eicosanol, n-Docosanol, n-Tetracosanol, n-Hexacosanol, n-Octacosanol, und/oder n-Triacontanol, sowie Gemische der vorstehend genannten Alkohole, beispielsweise NAFOL^{®} Typen wie NAFOL^{®} 22+ (Sasol).
- Oxoalkohole wie bespielsweise Isoheptanol, Isooctanol, Isononanol, Isodecanol, Isoundecanol, Isotridecanol (beispielsweise Exxal®-Typen 7, 8, 9, 10, 11, 13).
- Alkohole, die in 2-Stellung verzweigt sind; hierbei handelt es sich um die dem Fachmann bekannten Guerbetalkohole, die durch Dimerisierung von primären Alkoholen über die so genannte Guerbetreaktion zugänglich sind. Als besonders bevorzugte Alkohole seien hier genannt: Isofol^{®}12 (Sasol), Rilanit^{®}G16 (Cognis).
- Alkohole, die durch die Friedel-Crafts-Alkylierung mit oligomerisierten Olefinen erhalten werden und die dann neben einem gesaettigten Kohlenwasserstoffrest einen aromatischen Ring enthalten. Als besonders bevorzugte Alkohole seien hier genannt: i-Octylphenol und i-Nonylphenol.
- Alkohole der allgemeinen Formel (4) der EP 761780 A2, S.4 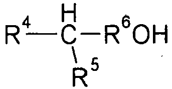 oder Alkohole der allgemeinen Formel (5) der EP 761780 A2, S.4 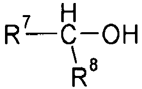 wobei
- R⁴, R⁵, R⁷ und R⁸ unabhängig voneinander die in EP 761780 A2, S.4, Zeilen 45 bis 58 beschriebene Bedeutung haben; bevorzugt sind R⁴, R⁵, R⁷ und R⁸ unabhängig voneinander Alkylreste mit wenigstens 4 Kohlenstoffatomen und die Gesamtzahl der Kohlenstoffatome der Alkohole beträgt höchstens 30,
- R⁶ ein Alkylenrest ist wie beispielsweise -CH₂-, -CH₂-CH₂-, -CH₂-CH(CH₃)-;

Beispielsweise sei hier 2-Decyl-1-tetradecanol als geeigneter Alkohol genannt.

In einer Ausführungsform werden Gemische ethoxylierter C₁₆-C₁₈-Fettalkohole verwendet, um Abschnitte T in die Polyurethane einzuführen.

In einer weiteren Ausführungsform werden ethoxylierte iso-C₁₃-Oxoalkohole oder Gemische davon verwendet, um Abschnitte T in die Polyurethane einzuführen.

In einer weiteren Ausführungsform werden Gemische umfassend ethoxylierte C₁₆-C₁₈-Fettalkohole und ethoxylierte iso-C₁₃-Oxoalkohole verwendet, um Abschnitte T in die Polyurethane einzuführen.

In einer weiteren Ausführungsform werden die zuvor beschriebenen Alkohole der allgemeinen Formel (4) oder (5) der EP 761780 A2, S.4, in ihrer ethoxylierten Form verwendet, um Abschnitte T in die Polyurethane einzuführen.

Selbstverständlich können zusätzlich auch andere Abschnitte T in die Polyurethane eingeführt werden.

Selbstverständlich können die hydrophoben Abschnitte T auch durch beliebige Mischungen der zuvor beschriebenen ethoxylierten Alkohole in die Polyurethane eingebracht werden.

Naturgemäß werden durch das erfindungsgemäße Verfahren, wie bei Polymerisationsreaktionen üblich, Mischungen von unterschiedlichen Polymeren, vorliegend also Mischungen verschiedener Polyurethane erhalten.
Der hier verwendete Begriff "Polyurethan" kann sowohl jedes einzelne Polyurethanmolekül als auch die Gesamtheit der durch die erfindungsgemäßen Verfahren erhältlichen Polyurethanmoleküle bezeichnen.

Bei den durch das erfindungsgemäße Herstellverfahren erhältlichen Polyurethanen handelt es sich bevorzugt um Gemische, welche die beschriebenen Polyurethanstrukturen enthalten.

Erfindungsgemäß ist demnach auch die Herstellung von Mischungen von Polyurethanen, deren endständige, hydrophobe Abschnitte T verzweigte und/oder unverzweigte Alkylreste sind. Erfindungsgemäß ist auch die Herstellung von Mischungen, die Polyurethane enthalten, die sowohl verzweigte als auch unverzweigte endständige, hydrophobe Abschnitte T enthalten.

Bevorzugt enthält wenigstens ein Teil der nach dem erfindungsgemäßen Verfahren erhältlichen Polyurethanmoleküle Allophanatsegmente. Gegenstand der Erfindung ist also auch ein erfindungsgemäßes Verfahren, wobei wenigstens ein Teil der erhaltenen Polyurethane AllophanatSegmente enthält.

Bevorzugt enthält wenigstens ein Teil der nach dem erfindungsgemäßen Verfahren erhältlichen Polyurethanmoleküle Isocyanuratsegmente.

Gegenstand der Erfindung ist also auch ein erfindungsgemäßes Verfahren, wobei wenigstens ein Teil der Polyurethane Isocyanurat-Segmente enthält.

### Hydrophile Abschnitte S

In den nach dem erfindungsgemäßen Verfahren erhältlichen Polyurethanen schließen sich an die terminalen hydrophoben Abschnitte T hydrophile Abschnitte S an.
In den Polyurethanen können die Abschnitte S unabhängig voneinander gleich oder verschieden, linear oder verzweigt sein.

In einer Ausführungsform sind die hydrophilen Abschnitte S von verschiedener Länge und linear.

Die Abschnitte S enthalten bevorzugt Reste von Alkylenoxiden. Bevorzugt liegt die Anzahl im Bereich von 2 bis 150 Alkylenoxidresten, besonders bevorzugt im Bereich von 5 bis 100 Alkylenoxidresten und ganz besonders bevorzugt im Bereich von 10 bis 100 Alkylenoxidresten.

Die hydrophilen Abschnitte S umfassen oder bestehen bevorzugt aus Ethylenoxidresten. Die hydrophilen Abschnitte S enthalten in einer bevorzugten Ausführungsform Ethylenoxidreste (EO-Einheiten), deren Anzahl im Bereich von 2 bis 150 EO-Einheiten liegt, besonders bevorzugt im Bereich von 5 bis 100 EO-Einheiten und ganz besonders bevorzugt im Bereich von 10 bis 100 EO-Einheiten.

In einer bevorzugten Ausführungsform bestehen die Abschnitte S aus 5 bis 30, bevorzugt 10 bis 25 EO-Einheiten

In einer anderen Ausführungsform bestehen die Abschnitte S aus 30 bis 100, bevorzugt 40 bis 100 EO-Einheiten.

Die Zahl der EO-Einheiten pro Molekül ethoxylierten Alkohols wird auch als Ethoxylierungsgrad bezeichnet.

Die im erfindungsgemäßen Verfahren bevorzugt eingesetzten ethoxylierten Alkohole haben einen Ethoxylierungsgrad, der im Bereich von 2 bis 150 Ethylenoxidresten liegt, vorzugsweise im Bereich von 5 bis 100 Ethylenoxidresten und weiter bevorzugt im Bereich von 10 bis 100 Ethylenoxidresten.

In einer besonders bevorzugten Ausführungsform haben die eingesetzten ethoxylierten Alkohole einen Ethoxylierungsgrad im Bereich von 10 bis 25 Ethylenoxidresten.

In einer Ausführungsform wird als mindestens einer der eingesetzten Alkohole eine verzweigte, nicht-ionische Verbindung der Strukturformel RO(CH₂CH₂O)ₓH , wobei R für einen C₁₃-Alkylrest, bevorzugt einen iso-C₁₃-Alkylrest steht, und mit x = 3, 5, 6, 6.5, 7, 8, 10, 12, 15 oder 20, bevorzugt x =10 verwendet. Kommerziell ist ein solcher ethoxylierter, alkylverzweigter Alkohol beispielsweise als Lutensol^{®}TO10 erhältlich.

In einer Ausführungsform wird als mindestens einer der eingesetzten Alkohole ein Alkohol der allgemeinen Formel (4) der EP 761780 A2, S.4 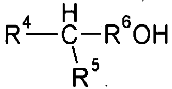 oder ein Alkohol der allgemeinen Formel (5) der EP 761780 A2, S.4 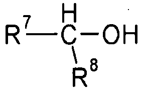 verwendet, wobei
- R⁴, R⁵, R⁷ und R⁸ unabhängig voneinander die in EP 761780 A2, S.4, Zeilen 45 bis 58 beschriebene Bedeutung haben; bevorzugt sind R⁴, R⁵, R⁷ und R⁸ unabhängig voneinander Alkylreste mit wenigstens 4 Kohlenstoffatomen und die Gesamtzahl der Kohlenstoffatome der Alkohole beträgt höchstens 30,
- R⁶ ein Alkylenrest ist wie beispielsweise -CH₂-, -CH₂-CH₂-, -CH₂-CH(CH₃)-;

Beispielsweise sei hier 2-Decyl-1-tetradecanol als geeigneter Alkohol genannt.

In einer Ausführungsform der Erfindung werden Mischungen aus ethoxylierten linearen und ethoxylierten verzweigten langkettigen Alkoholen, insbesondere Mischungen aus den vorgenannten Typen, eingesetzt.

In einer Ausführungsform der Erfindung umfassen die hydrophilen Abschnitte S Mischungen aus EO- und PO-Einheiten.

Ebenso können die Abschnitte S längerkettige Alkylenoxidreste enthalten, mit der Maßgabe, dass die Abschnitte S insgesamt hydrophil sein müssen. Die Hydrophilie kann beispielsweise über den Anteil an EO-Einheiten in den Abschnitten S gesteuert werden.

### Hydrophobe Abschnitte D

An jeden hydrophilen Abschnitt S schließt sich mindestens ein hydrophober Abschnitt D an. Dabei kann ein Abschnitt S auch im Molekülinnem der erfindungsgemäßen Polyurethane enthalten sein. In diesem Fall ist dieser Abschnitt S nicht wie ein randständiger Abschnitt S mit einem Abschnitt D und einem Abschnitt T, sondern auf mindestens zwei Seiten mit Abschnitten D verbunden. Bevorzugt ist ein Abschnitt S im Molekülinnern auf beiden Seiten mit je einem Abschnitt D verbunden.

Für alle randständigen Abschnitte S gilt, dass sie mit einem endständigen Abschnitt T direkt verbunden sind. Sollte ein Abschnitt S in geringem Maße verzweigt sein, so könnte er an zwei oder mehr Stellen mit hydrophoben Abschnitten D direkt verbunden sein. Bevorzugt schließt sich an jeden hydrophilen Abschnitt S auf mindestens einer Seite ein hydrophober Abschnitt D an.

In einer besonders bevorzugten Ausführungsform sind alle Abschnitte S unverzweigt und randständig und mit einem Abschnitt T auf der einen Seite und einem Abschnitt D auf der anderen Seite verbunden.

Durch das erfindungsgemäße Verfahren werden Polyurethanmoleküle erhalten, die mindestens zwei hydrophobe Abschnitte D enthalten. Je nachdem, wie das erfindungsgemäße Verfahren durchgeführt wird, werden daneben aber auch Polyurethanmoleküle erhalten, die nur einen hydrophoben Abschnitt D enthalten.

In den Polyurethanmolekülen mit mindestens zwei hydrophoben Abschnitten D können diese gleich oder unabhängig voneinander verschieden sein.

Die Abschnitte D können verzweigt mit kurzkettigen hydrophoben Verzweigungen oder unverzweigt sein. Bevorzugt ist wenigstens ein Teil der Abschnitte D verzweigt.
Bevorzugt enthalten die Abschnitte D wenigstens eine hydrophobe Kette von Kohlenstoffatomen, deren Länge im Bereich von 2 bis 20 Kohlenstoffatomen, bevorzugt 3 bis 16 Kohlenstoffatomen und insbesondere im Bereich von 4 bis 12 Kohlenstoffatomen liegt.

Bevorzugt enthalten die Abschnitte D Diisocyanatreste. Besonders bevorzugt enthalten die Abschnitte D Reste von aliphatischen Diisocyanaten. So kann beispielsweise ein hydrophober Abschnitt D aus einem oder mehreren aliphatischen Diisocyanatresten bestehen. Bevorzugt besteht ein Abschnitt D aus einem bis zehn aliphatischen Diisocyanatresten, besonders bevorzugt aus einem bis fünf aliphatischen Diisocyanatresten, ganz besonders bevorzugt enthält er ein, zwei oder drei aliphatische Diisocyanatreste.

Die hydrophoben Abschnitte D können aliphatische Diisocyanatreste mit langen, mittellangen oder kurzen aliphatischen Einheiten enthalten.

In einer der bevorzugten Ausführungsformen handelt es sich bei den Abschnitten D der nach dem erfindungsgemäßen Verfahren hergestellten Polyurethane um cycloaliphatische oder aliphatische Diisocyanatreste.

Besonders bevorzugt als Abschnitte D sind aliphatische Diisocyanatreste. Als aliphatische Diisocyanate seien beispielhaft genannt: 1,4-Butylendiisocyanat, 1,12-Dodecamethylendiisocyanat, 1,10-Decamethylendiisocyanat, 2-Butyl-2-ethylpentamethylendiisocyanat, 2,4,4- oder 2,2,4-Trimethylhexamethylendiisocyanat und insbesondere Hexamethylendiisocyanat (HDI).

Als cycloaliphatische Diisocyanate seien beispielhaft genannt: Isophorondiisocyanat (IPDI), 2-Isocyanatopropylcyclohexylisocyanat, 4-Methyl-cyclohexan-1,3-diisocyanat (H-TDI) und 1,3-Bis-(isocyanatomethyl)-cyclohexan.

Auch sogenannte H₁₂-MDI oder im Englischen "saturated MDI" genannte Diisocyanate, wie z.B. 4,4'-Methylen-bis-(cyclohexylisocyanat) (alternativ auch Dicyclohexylmethan-4,4'-diisocyanat genannt) oder 2,4'-Methylenbis(cyclohexyl)-diisocyanat können als Reste in Abschnitten D der erfindungsgemäßen Polyurethane PU enthalten sein.

Selbstverständlich kann man im erfindungsgemäßen Verfahren Mischungen der vorstehend genannten Diisocyanate einsetzen, um Mischungen unterschiedlicher Polyurethane herzustellen.

In einer bevorzugten Ausführungsform enthält ein Teil der nach dem erfindungsgemäßen Verfahren erhältlichen Polyurethane hydrophobe Abschnitte D mit Allophanat-Strukturen. Allophanat-Strukturen entstehen durch Addition einer Isocyanat-Gruppe an eine Urethan-Einheit.

In einer bevorzugten Ausführungsform enthält ein Teil der nach dem erfindungsgemäßen Verfahren hergestellten Polyurethane hydrophobe Abschnitte D mit Isocyanurat-Strukturen. Isocyanurat-Strukturen entstehen durch die Addition von 3 Isocyanatgruppen (Trimerisierung).

In einer weiteren bevorzugten Ausführungsform enthält ein Teil der nach dem erfindungsgemäßen Verfahren hergestellten Polyurethane sowohl hydrophobe Abschnitte D mit Allophanat-Strukturen als auch hydrophobe Abschnitte D mit Isocyanurat-Strukturen.

In einer weiteren Ausführungsform enthält ein Teil der nach dem erfindungsgemäßen Verfahren hergestellten Polyurethane hydrophobe Abschnitte D mit Biuret-Strukturen. Biuret-Strukturen entstehen durch die Addition einer Isocyanat-Gruppe an eine Harnstoff-Einheit. Harnstoffeinheiten wiederum entstehen durch Addition von primären Aminen an Isocyanatgruppen.

### Hydrophile Abschnitte P

Durch das erfindungsgemäße Verfahren werden Polyurethanmoleküle erhalten, die mindestens einen von den hydrophilen Abschnitten S verschiedenen hydrophilen Abschnitt P enthalten. Dabei gilt, dass sich an P mindestens zwei hydrophobe Abschnitte D anschließen. Die Abschnitte P der erfindungsgemäßen Polyurethane können unabhängig voneinander gleich oder verschieden sein.

Je nachdem, wie das erfindungsgemäße Verfahren durchgeführt wird, werden aber zusätzlich auch Polyurethanmoleküle erhalten, die keinen hydrophilen Abschnitt P enthalten.

Ist in einem erfindungsgemäßen Polyurethan mehr als ein Abschnitt P enthalten, so befindet sich zwischen den hydrophilen Abschnitten P jeweils mindestens ein hydrophober Abschnitt D. Sind in einem erfindungsgemäßen Polyurethan mehr als ein Abschnitt P enthalten, so können diese gleich oder verschieden sein.

Die erfindungsgemäßen Polyurethane können in einer Ausführungsform zwischen zwei hydrophilen Abschnitten P eine Sequenz von Abschnitten der Reihenfolge hydrophober Abschnitt D, dann hydrophiler Abschnitt S, dann wieder hydrophober Abschnitt D enthalten. Sind in einem erfindungsgemäßen Polyurethan also mehr als ein Abschnitt P enthalten, so können in einem solchen Fall die Abschnitte im Molekülinnem eine Abfolge von P-D-P oder von P-D-S-D-P aufweisen. Sollten mehr als zwei Abschnitte P enthalten sein, so sind beide Abfolgen in einem Molekül möglich.

Bevorzugt sind in einem Molekül der erfindungsgemäß erhältlichen Polyurethane nur ein oder zwei Abschnitte P enthalten.

Eingebracht werden die hydrophilen Abschnitte P in die Polyurethane bevorzugt durch die Verwendung von hydrophilen Polyolen. Diese enthalten pro Molekül mindestens zwei OH- Gruppen und mindestens zwei funktionelle Gruppen, die ausgewählt sind aus den Funktionen -O- (Ethergruppen) und -COO- (Estergruppen), wobei das Molgewicht dieser hydrophilen Verbindungen mindestens 300 und vorzugsweise mindestens 1200 beträgt.

Eine Ausführungsform der Erfindung ist ein erfindungsgemäßes Verfahren, dadurch gekennzeichnet, dass der mindestens eine hydrophile Abschnitt P ein zahlenmittleres Molekulargewicht von 1500 bis 20000 g/mol, bevorzugt von 4000 bis 12000 g/mol aufweist.

Geeignete hydrophile Polyole sind beispielsweise die Polymerisationprodukte des Ethylenoxids, deren Misch- oder Pfropfpolymerisationsprodukte sowie die durch Kondensation von mehrwertigen Alkoholen oder Mischung derselben und die durch Ethoxylierung von mehrwertigen Alkoholen, Amiden, Polyamiden und Aminoalkoholen gewonnenen Polyether. Beispiele dafür sind etwa Polyethylenglykole, Anlagerungsprodukte von Ethylenoxid an Trimethylolpropan, EO-PO-Blockcopolymere, OH-terminierte Polyester wie beispielsweise solche vom Typ der mehrfunktionellen Polycaprolactone.

Bevorzugte hydrophile Polyole sind Polyetherpolyole. Dies sind solche hydrophilen Polyole, die pro Molekül mindestens zwei OH-Gruppen und mindestens zwei Funktionen -O- (Ethergruppen) enthalten. Diese Polyetherpolyole sind in aller Regel so stark hydrophil, dass sie bei Raumtemperatur (20°C) wasserlöslich sind.

Zur Herstellung der Polyurethane nach dem erfindungsgemäßen Verfahren eignen sich bevorzugt solche Polyetherpolyole, die überwiegend Polyethylenglykol enthalten. Besonders bevorzugt ist es, wenn diese Polyethylenglykole pro Molekül eine mittlere Menge von EO-Einheiten im Bereich von 30 bis 450 aufweisen.

Bevorzugt sind Polyole der allgemeinen Formel HO-(CH₂-CH₂-O)ₙ-H, wobei n die Werte 30 bis 450 annehmen kann. Hierbei handelt es sich um Polyethylenglykole, die Kondensationsprodukte des Ethylenoxids mit Ethylenglykol oder Wasser darstellen.
Vorzugsweise stellt man das Molgewicht dieser Polyethylenglykole auf Werte im Bereich von 1500 bis 20000 g/mol ein.

Es können aber auch EO-PO-Blockcopolymere verwendet werden, um die Abschnitte P in die erfindungsgemäß erhältlichen Polyurethane einzubringen. Beispielsweise können EO-PO-Blockcopolymere der allgemeinen Formel HO-(EO)ₘ-(PO)ₙ-(EO)ₒ-H verwendet werden, wobei m und o unabhängig voneinander ganze Zahlen im Bereich von 10 bis 100, vorzugsweise von 20 bis 80 bedeuten, n eine ganze Zahl im Bereich von 5 bis 50, bevorzugt von 20 bis 40 bedeutet und wobei m, n und o so gewählt werden, dass HO-(EO)ₘ-(PO)ₙ-(EO)ₒ-H wasserlöslich ist.

Erfindungsgemäß weisen die im Wesentlichen linearen Polyetherreste, die die Abschnitte P bilden, bevorzugt ein zahlenmittleres Molekulargewicht Mₙ von mindestens 1500 g/mol und höchstens 20000 g/mol auf.

In einer bevorzugten Ausführungsform haben die im Wesentlichen linearen Polyetherreste zahlenmittlere Molekulargewichte im Bereich von 1500 g/mol bis 15000 g/mol.

In einer weiter bevorzugten Ausführungsform haben die im Wesentlichen linearen Polyetherreste zahlenmittlere Molekulargewichte im Bereich von 4000 g/mol bis 12000 g/mol.

In einer besonders bevorzugten Ausführungsform haben die im Wesentlichen linearen Polyetherreste zahlenmittlere Molekulargewichte im Bereich von 6000 g/mol bis 10000 g/mol.

In einer besonders bevorzugten Ausführungsform haben die linearen Polyetherreste ein zahlenmittleres Molekulargewicht Mₙ von etwa 10000 g/mol.

In einer weiteren besonders bevorzugten Ausführungsform haben die linearen Polyetherreste ein zahlenmittleres Molekulargewicht Mₙ von etwa 6000 g/mol.

Sämtliche hydrophilen Abschnitte der nach dem erfindungsgemäßen Verfahren erhältlichen Polyurethane, d.h. die Abschnitte S und P, können Polyetherreste sein.

In einer bevorzugten Ausführungsform bestehen die hydrophilen Abschnitte der erfindungsgemäßen Polyurethane aus
- Polyalkylenoxid-Einheiten (Abschnitte P) und
- Polyethylenoxid-Einheiten (Abschnitte S).

In einer besonders bevorzugten Ausführungsform der nach dem erfindungsgemäßen Verfahren erhältlichen Polyurethane bestehen alle Abschnitte P und S aus EO-Einheiten.

Durch das erfindungsgemäße Verfahren werden Polyurethanmoleküle erhalten, die mindestens drei hydrophile Abschnitte enthalten. In einer der bevorzugten Ausführungsformen sind dies zwei Abschnitte S und mindestens ein Abschnitt P.

Je nach Reaktionsführung werden zusätzlich aber auch Polyurethanmoleküle mit nur zwei hydrophilen Abschnitten S und ohne hydrophilen Abschnitt P erhalten.

Erfindungsgemäß bevorzugt werden durch das erfindungsgemäße Verfahren also auch Polyurethane erhalten, die
I) mindestens zwei hydrophile Abschnitte S,
II) keinen hydrophilen Abschnitt P,
III) mindestens zwei endständige hydrophobe Abschnitte T,
IV) mindestens einen von T verschiedenen hydrophoben Abschnitt D umfassen,
   wobei
   a) sich an jeden Abschnitt T ein Abschnitt S unmittelbar anschließt,
   b) sich an jeden Abschnitt S ein Abschnitt D anschließt.

Die durch das erfindungsgemäße Verfahren hergestellten Polyurethane, die zusätzlich Allophanatstrukturen enthalten, umfassen bevorzugt mindestens drei Abschnitte S und mindestens einen Abschnitt P.

Je nach Reaktionsführung werden aber zusätzlich auch Polyurethane erhalten, die Allophanatstrukturen und mindestens drei Abschnitte S, jedoch keinen Abschnitt P enthalten.

Nach dem erfindungsgemäßen Verfahren hergestellte Polyurethane, die zusätzlich Isocyanuratstrukturen enthalten, umfassen bevorzugt mindestens drei Abschnitte S und mindestens einen Abschnitt P.

Je nach Reaktionsführung werden aber zusätzlich auch Polyurethane erhalten, die Isocyanuratstrukturen und mindestens drei Abschnitte S, jedoch keinen Abschnitt P enthalten.

Wenigstens ein Teil der nach dem erfindungsgemäßen Verfahren hergestellten Polyurethane sind linear und weisen folgende Abfolge der Abschnitte auf: T-S-D-P-D-S-T oder T-S-D-P-D-P-D-S-T. Je nach Reaktionsführung werden zusätzlich auch Polyurethane erhalten, die die Abfolge T-S-D-S-T aufweisen.

In einer bevorzugten Ausführungsform der Erfindung enthält wenigstens ein Teil der nach dem erfindungsgemäßen Verfahren hergestellten Polyurethane Allophanat und/oder Isocyanuratstrukturen und weist folgende Abfolge der Abschnitte auf:

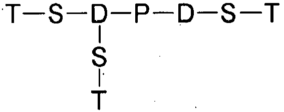

Beispielsweise können nach dem erfindungsgemäßen Verfahren hergestellte Polyurethane, die zusätzlich Allophanatstrukturen enthalten, folgende Struktur aufweisen:

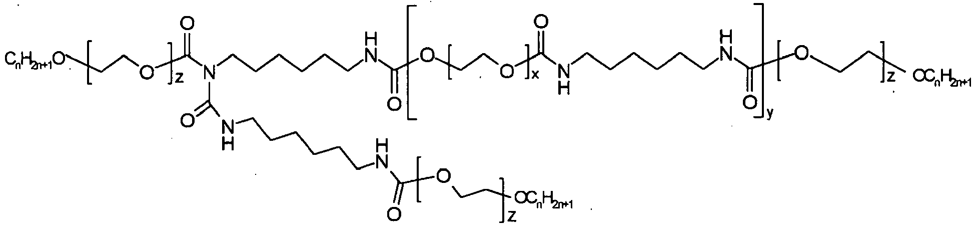

Beispielsweise können nach dem erfindungsgemäßen Verfahren hergestellte Polyurethane, die zusätzlich Isocyanuratstrukturen enthalten, folgende Struktur aufweisen:

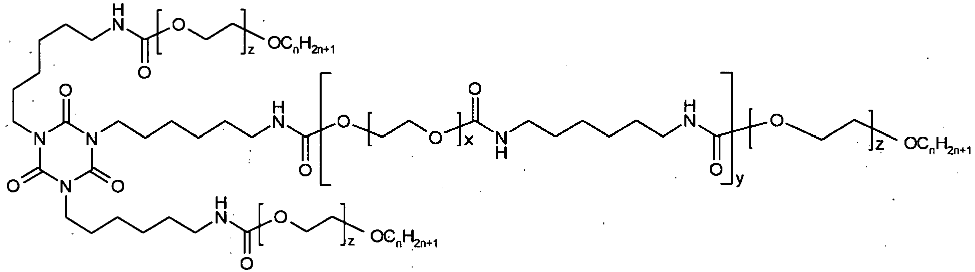

Für jeden Abschnitt P gilt, dass sein Molekulargewicht größer ist als das jedes im gleichen Molekül enthaltenen Abschnitts S.

Das Verhältnis der Molekulargewichte Mₙ eines jeden hydrophilen Abschnittes S der erfindungsgemäßen Polyurethane zu dem Molekulargewicht eines jeden hydrophilen Abschnittes P liegt im Bereich von 1 zu 1,4 bis 1 zu 140, bevorzugt im Bereich von 1 zu 1,7 bis 1 zu 120.

In einer bevorzugten Ausführungsform ist das Verhältnis gleich 1 zu x, wobei x gleich oder größer 2 ist, bevorzugt gleich oder größer 2,3 und besonders bevorzugt ist x gleich oder größer 2,8. Besonders bevorzugt liegt das Verhältnis im Bereich von 1 zu 2,8 bis 1 zu 115, ganz besonders bevorzugt im Bereich von 1 zu 3 bis 1 zu 95 und insbesondere bevorzugt im Bereich von 1 zu 3,4 bis 1 zu 80.

Durch das erfindungsgemäße Verfahren werden in der Regel Mischungen verschiedener Polyurethane erhalten. Eine derartige Mischung kann z.B. Polyurethane enthalten, die zwar die gleiche Abfolge der Abschnitte T, S, D und / oder P aufweisen, wobei sie sich aber in mindestens einem der Abschnitte strukturell voneinander unterscheiden. Als Beispiel hierfür sei ein unterschiedlicher Abschnittsaufbau oder eine unterschiedliche Abschnittskettenlänge genannt. So können in einer Mischung der nach dem erfindungsgemäßen Verfahren hergestellten Polyurethane die Abschnitte T verschieden sein. Beispielsweise kann eine erfindungsgemäße Mischung Polyurethane enthalten, deren Abschnitte T alle verzweigt sind, und/oder solche, deren Abschnitte T alle linear sind, und/oder solche Polyurethane, die sowohl wenigstens einen linearen Abschnitt T als auch wenigstens einen verzweigten Abschnitt T enthalten.

In einer Ausführungsform ist die Summe der Molekulargewichte aller Abschnitte T, plus der Molekulargewichte von Abschnitten D kleiner oder gleich der Summe der Molekulargewichte aller Abschnitte P zu halten.

### Katalysator

Zur Herstellung der Polyurethane nach dem erfindungsgemäßen Verfahren werden als Katalysator Carbonsäuresalze von Alkalimetallen, Carbonsäuresalze von Erdalkalimetallen oder Mischungen davon verwendet.

Die Carbonsäuren, deren (Erd)alkalimetallsalze als Katalysatoren im erfindungsgemäßen Verfahren verwendet werden, sind bevorzugt Monocarbönsäuren der allgemeinen Formel R-COOH, wobei R ein beliebiger organischer, beispielsweise ein aliphatischer, ein aromatischer oder ein heterozyklischer Rest sein kann.

Bevorzugt ist R ein aliphatischer Rest, also beispielsweise ein Alkyl-, ein Alkenyl- oder ein Alkinylrest. R kann auch Heteroatome enthalten, beispielsweise kann es sich bei der Carbonsäure also um eine Hydroxycarbonsäure handeln.

In einer Ausführungsform der Erfindung ist R ein Kohlenwasserstoffrest mit 1 bis 20, bevorzugt mit 1 bis 12 Kohlenstoffatomen. R kann linear oder verzweigt, gesättigt oder ungesättigt sein. In einer Ausführungsform der Erfindung ist die Carbonsäure Essigsäure.
In einer weiteren Ausführungsform der Erfindung ist die Carbonsäure Octansäure.
In einer weiteren Ausführungsform der Erfindung ist die Carbonsäure 2-Ethyl-Hexansäure.
In einer weiteren Ausführungsform der Erfindung ist die Carbonsäure Neodecansäure.
In einer weiteren Ausführungsform der Erfindung ist die Carbonsäure n-Decansäure.
In einer weiteren Ausführungsform der Erfindung ist die Carbonsäure Stearinsäure.
In einer weiteren Ausführungsform der Erfindung ist die Carbonsäure Ricinolsäure ((9Z,12R)-12-Hydroxy-9-octadecensäure).

In einer weiteren Ausführungsform der Erfindung ist die Carbonsäure eine Hydroxycarbonsäure wie beispielsweise Citronensäure oder Milchsäure, insbesondere Milchsäure.

Wird als Katalysator wenigstens ein Carbonsäuresalz eines Alkalimetalls gewählt, so wird das Alkalimetall bevorzugt ausgewählt aus Natrium und Kalium, besonders bevorzugt Kalium.

Wird als Katalysator wenigstens ein Carbonsäuresalz eines Erdalkalimetalls gewählt, so wird das Erdalkalimetall bevorzugt ausgewählt aus Calcium und Magnesium, besonders bevorzugt Calcium.

In einer bevorzugten Ausführungsform der Erfindung wird Kaliumacetat als Katalysator für die Polyurethan-Herstellung nach dem erfindungsgemäßen Verfahren verwendet.

In einer bevorzugten Ausführungsform der Erfindung wird Kaliumlactat als Katalysator für die Polyurethan-Herstellung nach dem erfindungsgemäßen Verfahren verwendet.

Selbstverständlich können auch Gemische aus zwei oder mehreren Carbonsäuresalzen von (Erd)alkalimetallen als Katalysatoren zur Herstellung von erfindungsgemäßen Polyurethanen PU eingesetzt werden.

In einer bevorzugten Ausführungsform wird ein Katalysator verwendet.

Zusätzlich zu diesen Katalysatoren können weitere, dem Fachmann auf dem Gebiet der Polyurethanherstellung bekannte Katalysatoren eingesetzt werden.

Solche üblicherweise in der Polyurethanchemie verwendeten Katalysatoren sind organische Amine, insbesondere tertiäre aliphatische, cycloaliphatische oder aromatische Amine, und Lewis-saure organische Metallverbindungen.

Als Lewis-saure organische Metallverbindungen kommen z.B. Metallkomplexe wie Acetylacetonate des Eisens, Titans, Zinks, Aluminiums, Cobalts, Mangans, Nickels und Zirkons in Betracht wie z.B. Zirkon-2,2,6,6-tetramethyl-3,5-heptandionat. Weitere geeignete Metallverbindungen werden von Blank et al. in Progress in Organic Coatings, 1999, 35, 19 ff. beschrieben.

Auch Wismut-, Cobalt- oder Zinkkatalysatoren sowie Cäsium- oder Titansalze können als Katalysatoren eingesetzt werden.

In einer Ausführungsform der Erfindung beträgt die Menge an solchen weiteren Katalysatoren, die keine Carbonsäuresalze von Alkalimetallen, Carbonsäuresalze von Erdalkalimetallen oder Mischungen davon sind, höchstens 10 Gew.-%, bevorzugt höchstens 5 Gew.-%, besonders bevorzugt höchstens 1 Gew.% und insbesondere höchstens 0,1 Gew.-% der Gesamtmenge an Katalysator.

Eine Ausführungsform der Erfindung ist das erfindungsgemäße Verfahren, das dadurch gekennzeichnet ist, dass die Herstellung in Gegenwart von weniger als 10 ppm Zinn erfolgt.

In einer weiteren Ausführungsform der Erfindung ist das erfindungsgemäße Verfahren dadurch gekennzeichnet, dass die Herstellung in Gegenwart von weniger als 10 ppm Zink erfolgt.

In einer weiteren Ausführungsform der Erfindung werden außer dem mindestens einen Carbonsäuresalz mindestens eines Metalls ausgewählt aus Alkalimetallen, Erdalkalimetallen und deren Mischungen keine weiteren Katalysatoren zur Herstellung der Polyurethane verwendet.

Man setzt den Katalysator oder das Gemisch von Katalysatoren vorzugsweise in einer Menge im Bereich von 50 ppm bis 5000 ppm bezogen auf das Gesamtgewicht aller reagierenden Verbindungen ein. Bevorzugt wird der Katalysator in einer Menge im Bereich von 50 bis 2500 ppm, besonders bevorzugt in einer Menge im Bereich von 100 bis 1000 ppm, bezogen auf das Gesamtgewicht aller reagierenden Verbindungen, eingesetzt.

Man kann - je nach Beschaffenheit des Katalysators - den Katalysator der Reaktionsmischung in fester oder flüssiger Form zusetzen. Geeignete Lösemittel sind nicht-wässrige Lösungsmittel wie beispielsweise aromatische oder aliphatische Kohlenwasserstoffe, u.a. Toluol, Xylol, Ethylacetat, Hexan und Cyclohexan, sowie Carbonsäureester wie beispielsweise Ethylacetat. Weiterhin geeignete Lösemittel sind Aceton, THF, DMSO, DMF, DMAc und N-Methylpyrrolidon und N-Ethylpyrrolidon.

Bevorzugt setzt man den Katalysator / das Katalysatorgemisch in gelöster Form ein, besonders bevorzugt gelöst in den Polyetherpolyolen, mit denen die hydrophilen Abschnitte P in die Polyurethane eingebracht werden.

Der Katalysator kann wenigstens teilweise bereits in den für das erfindungsgemäße Verfahren verwendeten Polyetherpolyolen enthalten sein, sofern bei deren Herstellung Carbonsäuresalze von (Erd)alkalimetallen eingesetzt wurden oder entstanden sind.

In einer Ausführungsform der Erfindung umfassen die im erfindungsgemäßen Verfahren eingesetzten Polyetherdiole also wenigstens einen Teil, gegebenenfalls die gesamte benötigte Menge des Katalysators.

In einer Ausführungsform der Erfindung umfassen die im erfindungsgemäßen Verfahren eingesetzten Polyetherdiole vor Beginn des Verfahrens bereits wenigstens einen Teil des benötigten Katalysators und der verbleibende Teil wird zur Durchführung des Verfahrens zugegeben.

Bei dem erfindungsgemäßen Verfahren zur Herstellung der Polyurethane werden bevorzugt folgende Ausgangsprodukte verwendet:
A) Verbindungen, die die hydrophilen Abschnitte P in die Polyurethane einbringen: bevorzugt Polyole der allgemeinen Formel HO-(CH₂-CH₂-O)ₙ-H, wobei n bevorzugt die Werte 30 bis 450 annimmt; hierbei handelt es sich um Polyethylenglykole, die Kondensationsprodukte des Ethylenoxids mit Ethylenglykol oder Wasser darstellen. Bevorzugte Polyethylenglykole besitzen ein zahlenmittleres Molgewicht im Bereich von 6000 bis 12000 g/mol, besonders bevorzugte besitzen ein zahlenmittleres Molgewicht von 6000 bis 10000 g/mol.
B) Verbindungen, die die terminalen hydrophoben Abschnitte T und die den Abschnitten T jeweils benachbarten hydrophilen Abschnitte S einbringen:
   bevorzugt ethoxylierte C₁₆-C₁₈-Fettalkohole, ethoxylierte iso-C₁₃-Oxoalkohole, ethoxylierte verzweigte Alkohole gemäß der Production Examples 1 bis 24 der EP 761780 A2 und Mischungen daraus;
C) Verbindungen, die die hydrophoben Abschnitte D einbringen: aliphatische Diisocyanate, insbesondere Hexamethylendiisocyanat (HDI).

Das erfindungsgemäße Verfahren zur Herstellung der Polyurethane enthält in einer Ausführungsform die folgenden Schritte:
I) Herstellung einer Mischung enthaltend
   a. mindestens ein Polyetherdiol mit einem Molekulargewicht im Bereich von 6000 bis 12000 g/mol,
   b. mindestens ein alkylverzweigtes C₈-C₃₀-, bevorzugt C₁₂-C₃₀-Alkanol, das mit 10 bis 100 mol, bevorzugt mit 10 bis 40 mol, weiter bevorzugt mit 10 bis 25 mol Ethylenoxid pro mol Alkanol ethoxyliert ist,
   c. mindestens ein Carbonsäuresalz mindestens eines Alkali- oder Erdalkalimetalls, bevorzugt ein Kaliumcarboxylat,
II) gegebenenfalls Erwärmen der Mischung aus Schritt I) auf 60 bis 120°C, bevorzugt auf 80 bis 100°C;
III) gegebenenfalls Verminderung des Wassergehalts der Mischung bis zu einem Wassergehalt von höchstens 1000 ppm, bevorzugt höchstens 300 ppm, jeweils bezogen auf das Gesamtgewicht der Mischung;
IV) Zugabe mindestens eines Diisocyanates, bevorzugt Hexamethylendiisocyanat, zur Mischung;
V) Reagierenlassen der erhaltenen Reaktionsmischung bis zu einem Isocyanat-Gehalt von höchstens 0,1 Gew.-%, bezogen auf das Gesamtgewicht der Reaktionsmischung.

Unter Reaktionsmischung wird die Gesamtheit aller Substanzen verstanden, die im Reaktionsraum nach dem Zeitpunkt vorhanden ist, an dem die gesamte Menge an Katalysatoren, Isocyanaten, gegenüber Isocyanaten reaktiven Substanzen und allen weiteren Substanzen, wie beispielsweise Lösungsmitteln, dem Reaktionsraum vollständig zugeführt worden sind.

Unter Lösungsmitteln werden bei 20°C und 1 bar Druck flüssige Phasen verstanden, in denen eines oder mehrere der Ausgangsprodukte für die Polyurethane, also der Substanzen, die die hydrophilen und/oder hydrophoben Abschnitte in die Polyurethane einbringen oder die als Katalysator wirken, bei 20°C und 1 bar löslich sind.

In einer Ausführungsform der Erfindung liegt die Menge an Lösungsmitteln, die unterschiedlich sind von den Substanzen, die die hydrophilen und hydrophoben Abschnitte in die Polyurethane einbringen oder die als Katalysator wirken, im Bereich von 0 bis 10 Gew.-%, weiter bevorzugt von 0 bis 5 Gew.-% und insbesondere 0 bis 1 Gew.-%, jeweils bezogen auf das Gewicht der Reaktionsmischung.

In einer Ausführungsform der Erfindung wird das erfindungsgemäße Verfahren durchgeführt im Wesentlichen in Abwesenheit von Lösungsmitteln, die unterschiedlich sind von den Substanzen, die die hydrophilen und/oder hydrophoben Abschnitte in die Polyurethane einbringen oder die als Katalysator wirken.

In einer Ausführungsform der Erfindung beträgt die Menge an solchen Substanzen, die nach vollständiger Zugabe aller Substanzen während der Reaktion im Reaktionsraum vorhandenen sind und weder Katalysatoren, noch Substanzen, die in die entstehenden Polyurethane eingebaut werden und dadurch die hydrophilen und hydrophoben Abschnitte in die Polyurethane einbringen, noch Reaktionsprodukte sind, höchstens 10 Gew.-%, bevorzugt höchstens 5 Gew.-%, weiter bevorzugt höchstens 1 Gew.-% und besonders bevorzugt höchstens 0,1 Gew.-% der Gesamtmenge aller während der Reaktion im Reaktionsraum vorhandenen Substanzen.

Vereinfacht ausgedrückt, enthält die Reaktionsmischung in einer bevorzugten Ausführungsform außer den Substanzen, die die hydrophilen bzw. hydrophoben Abschnitte in die Polyurethane einbringen, den Reaktionsprodukten, gegebenenfalls geringen Mengen von Wasser und den Katalysatoren keine weiteren Substanzen.

Das bevorzugte Verfahren, bei dem außer den Ausgangsprodukten für die Polyurethanbildung keine weiteren Lösungsmittel eingesetzt werden, führt zu einer schnelleren Reaktion und Lösungsmittel müssen nicht abgetrennt werden.

Ein weiterer Vorteil des Verzichts auf Lösungsmittel, insbesondere auf organische Lösungsmittel, die unterschiedlich sind von den Substanzen, die die hydrophilen bzw. hydrophoben Abschnitte in die Polyurethane einbringen oder die als Katalysator wirken, ist die bessere Akzeptanz der resultierenden Polyurethane als Inhaltsstoffe kosmetischer Zubereitungen.

Sofern im erfindungsgemäßen Verfahren doch Lösungsmittel verwendet werden, die unterschiedlich sind von den Substanzen, die die hydrophilen bzw. hydrophoben Abschnitte in die Polyurethane einbringen oder die als Katalysator wirken, so werden diese nach der Reaktion möglichst weitgehend entfernt. "Nach der Reaktion" bedeutet den Zeitpunkt, zu dem der Gehalt an Isocyanatgruppen höchstens noch 0,1 Gew.-%, bezogen auf das Gesamtgewicht der Reaktionsmischung, beträgt.

### Einstufiges Verfahren

In einer bevorzugten Ausführungsform wird das Verfahren als einstufiges Verfahren durchgeführt.

"Einstufig" bedeutet hier, dass die Substanzen mit gegenüber Isocyanatgruppen reaktiven Gruppen im Wesentlichen gemeinsam und gleichzeitig mit den Isocyanatgruppen tragenden Substanzen in Kontakt gebracht werden.

In einer bevorzugten Ausführungsform der Erfindung wird also eine Mischung der Substanzen mit gegenüber Isocyanatgruppen reaktiven Gruppen mit den Isocyanatgruppen tragenden Substanzen in Kontakt gebracht.

Dabei kann bereits zu Beginn der Reaktion im Reaktionsraum die Gesamtmenge aller Substanzen mit gegenüber Isocyanatgruppen reaktiven Gruppen mit den Isocyanatgruppen tragenden Substanzen in Kontakt gebracht werden. Eine solche Vorgehensweise ist beispielsweise beschrieben im Beispiel 10 der EP 761780 A2 auf S.14, Zeilen 29 bis 47 und den folgenden Beispielen 11 bis 24.

Ein Kennzeichen des einstufigen Verfahrens ist, dass alle Arten von Substanzen mit gegenüber Isocyanatgruppen reaktiven Gruppen zu jedem Zeitpunkt der Reaktion nebeneinander vorliegen. Es ist aber nicht zwingend, dass das Mengenverhältnis der verschiedenen Substanzen in der Mischung konstant ist.

Im Unterschied zur zweistufigen Reaktion wird bei der einstufigen Reaktion nicht zunächst das Polyol mit einem Überschuss Polyisocyanat in Kontakt gebracht, so dass Präpolymere der Struktur -D-P-D- und -D-P-D-P-D- mit Isocyanat-Kettenenden entstehen und diese im zweiten Schritt mit dem ethoxylierten Alkohol umgesetzt werden, so dass dann die Strukturen T-S-D-P-D-S-T oder T-S-D-P-D-P-D-S-T entstehen.

Bei der bevorzugten einstufigen Reaktion werden von Anfang an neben Strukturen T-S-D-P-D-S-T und T-S-D-P-D-P-D-S-T auch Strukturen T-S-D-S-T gebildet.

Es ist für das einstufige Verfahren aber nicht zwingend, dass bereits zu Beginn der Reaktion im Reaktionsraum die Gesamtmenge aller Substanzen mit gegenüber Isocyanatgruppen reaktiven Gruppen vorliegt. Es ist auch denkbar, dass zu Beginn der Reaktion im Reaktionsraum nur jeweils ein Teil jeder Substanz mit gegenüber Isocyanatgruppen reaktiven Gruppen vorliegt und der jeweilige Rest der Substanzen während der Reaktion zugegeben wird.

Beim zweistufigen Verfahren wird in der ersten Stufe der überwiegende Anteil der Substanzen mit mehreren gegenüber Isocyanat reaktiven Gruppen mit den Isocyanatgruppen tragenden Substanzen in Kontakt gebracht und die Substanzen mit nur einer gegenüber Isocyanatgruppen reaktiven Gruppe reagieren in der zweiten Stufe mit den verbleibenden Isocyanatgruppen und bilden die Kettenenden der Polyurethane.

Beim einstufigen Verfahren ist die Menge von Polymeren des Aufbaus T-S-D-S-T größer als im zweistufigen Verfahren, während beim zweistufigen Verfahren die Menge an Polymeren des Aufbaus T-S-D-P-D-S-T größer ist.

Die durch das erfindungsgemäße Verfahren hergestellten Polyurethane werden in einer bevorzugten Ausführungsform in eine wässrige Phase überführt, gegebenenfalls nachdem Lösungsmittel abgetrennt wurden.

Es ist vorteilhaft, die nach der Überführung des Reaktionsproduktes in Wasser erhaltene wässrige Mischung zu stabilisieren.

Als Radikalfänger eignen sich beispielsweise Hydroxy-TEMPO, 2,6-Di-tert.-butyl-p-Kresol (Kerobit^{®}TBK), Hydrochinonmonomethylether, Tocopherol und Gemische dieser Verbindungen. Diese Stabilisatoren werden in einer Menge im Bereich von 5-500 ppm, bezogen auf das Gewicht der wässrigen Mischung, eingesetzt.

Weiterhin ist es vorteilhaft, Konservierungsmittel zu verwenden. Geeignet sind beispielsweise Phenoxyethanol, Methylisothiazolinon, Ethylhexylglycerin, 3-Acetyl-6-methyl-2H-pyran-2,4(3H)-dion, Benzoesäure, Parabene und Mischungen dieser Substanzen. Die Konservierungsmittel werden verwendet in Mengen im Bereich von 0,1 bis 1 Gew.-%, bezogen auf die wässrige Mischung.

Bevorzugt werden im erfindungsgemäßen Verfahren Substanzen mit gegenüber Isocyanatgruppen reaktiven Gruppen eingesetzt, die im Wesentlichen wasserfrei sind, um eine konkurrierende Reaktion der Isocyanatgruppen mit Wasser zu verhindern. Das Entfernen des Wassers aus diesen Substanzen kann im erfindungsgemäßen Verfahren durch azeotrope Destillation, Trocknen unter Vakuum oder andere dem Fachmann bekannte Methoden erfolgen. Beispielsweise wird das Wasser bis zu einem Wassergehalt der Substanzen von höchstens 500 ppm, bevorzugt höchstens 300 ppm entfernt.

Die Vorbereitung der eigentlichen Reaktion kann beispielsweise dergestalt sein, dass die Substanzen mit gegenüber Isocyanatgruppen reaktiven Gruppen vermindertem Druck, bevorzugt Vakuum, ausgesetzt werden und so das Wasser weitgehend entfernt wird.

Die Wasser enthaltenden Substanzen können auch mit einem Lösungsmittel wie Xylol, Toluol oder Aceton vermischt und das Wasser gemeinsam mit dem zugesetzten Lösungsmittel durch azeotrope Destillation entfernt werden.

Beim erfindungsgemäßen Verfahren kann das Verhältnis (mol zu mol) der eingesetzten Polyetherdiole zu eingesetzten Diisocyanaten im Bereich von 1 zu 1,1 bis 1 zu 2 liegen. Bevorzugt liegt das Verhältnis im Bereich von 1 zu 1,1 bis 1 zu 1,9. Besonders bevorzugt liegt das Verhältnis im Bereich von 1 zu 1,1 bis 1 zu 1,8. Insbesonders bevorzugt liegt das Verhältnis im Bereich von 1 zu 1,2 bis 1 zu 1,75. Selbstverständlich kann das Verhältnis auch 1 zu x mit x größer oder gleich 1,3, bevorzugt x größer oder gleich 1,5, sein.

In einer Ausführungsform ergibt sich daraus, dass in einem Molekül der erfindungsgemäß erhältlichen Polyurethane bevorzugt nur ein oder zwei Abschnitte P enthalten sind.

In einer Ausführungsform des erfindungsgemäßen Verfahrens wird zusätzlich zu den genannten Bereichen des Verhältnisses von Polyetherdiolen zu Diisocyanaten zusätzlich das Verhältnis von Polyetherdiolen zu ethoxylierten Alkoholen so gewählt, dass das molare Mengenverhältnis von eingesetzten Polyetherdiolen zu eingesetzten ethoxylierten Alkoholen im Bereich von 5 zu 1 bis 1 zu 2 liegt. Bevorzugt liegt dieses Verhältnis im Bereich von 2 zu 1 bis 1 zu 2, besonders bevorzugt im Bereich von 1,5 zu 1 bis 1 zu 2 und am meisten bevorzugt bei etwa 1 zu 1.

Für ein besonders bevorzugtes erfindungsgemäßes Verfahrens gilt, dass ein molares Mengenverhältnis von Polyetherdiolen zu Diisocyanaten zu ethoxylierten Alkoholen von 1 zu 1,2 bis1,75 zu 1 bis 2 und insbesondere von 1 zu 1,5 zu 1 eingesetzt wird.

Gegenstand der Erfindung ist auch das erfindungsgemäße Verfahren, dadurch gekennzeichnet, dass zur Herstellung der Polyurethane jeweils mindestens ein alkoxylierter C₄-C₃₀-Alkohol, ein Polyetherdiol und ein Diisocyanat eingesetzt werden.

Bevorzugt ist ein solches Verfahren, das dadurch gekennzeichnet ist, dass wenigstens ein C₄-C₃₀-Alkohol ein verzweigtes C₁₂-C₃₀-Alkanol ist, wenigstens ein Polyetherdiol ein Molekulargewicht Mₙ im Bereich von 4000 bis 12000 g/mol aufweist und wenigstens ein Diisocyanat ein aliphatisches Diisocyanat ist.

Gegenstand der Erfindung ist auch die Verwendung der erfindungsgemäßen Polyurethane zur Herstellung von wässrigen Zubereitungen. Bevorzugt werden dabei Zubereitungen, die mindestens 5 Gew.-%, insbesondere mindestens 20 Gew.-%, ganz besonders bevorzugt mindestens 30 Gew.-% und am meisten bevorzugt mindestens 70 Gew.-% Wasser enthalten.

Bevorzugt werden Zubereitungen, die höchstens 95 Gew.-%, besonders bevorzugt höchstens 90 Gew.-% und insbesondere höchstens 85 Gew.-% Wasser enthalten.

Bei den Wasser enthaltenden Zubereitungen kann es sich beispielsweise um Lösungen, Emulsionen, Suspensionen oder Dispersionen handeln.

Zusätzlich zu den durch das erfindungsgemäße Verfahren erhältlichen Polyurethanen können weitere Stoffe zur Herstellung der Zubereitungen verwendet werden, wie z.B. übliche Hilfsstoffe (beispielsweise Dispergiermittel und /oder Stabilisatoren), Tenside, Konservierungsstoffe, Antischaummittel, Duftstoffe, Netzmittel, UV-Filter, Pigmente, Emollients, Wirkstoffe, weitere Verdicker, Farbstoffe, Weichmacher, Feuchthaltemittel und/oder andere Polymere.

Die nach dem erfindungsgemäßen Verfahren erhältlichen Polyurethane und deren Mischungen werden bevorzugt dazu verwendet, Zubereitungen mit einem Gehalt an Salzen und Pigmenten von mehr als 1 Gew.-%, bezogen auf die Zubereitung, wirksam und stabil zu verdicken. "Stabil" bedeutet hierbei die Aufrechterhaltung einer gegenüber dem unverdickten Zustand erhöhten Viskosität über einen Zeitraum von mehreren Wochen und/oder bei Erhöhung der Temperatur der Zubereitung, beispielsweise auf bis zu 50°C.

Die nach dem erfindungsgemäßen Verfahren erhältlichen Polyurethane zeigen ihre verdickende Wirkung auch bei höheren Temperaturen bis etwa 50°C.

Weiterhin zeigen die nach dem erfindungsgemäßen Verfahren erhältlichen Polyurethane in einem breiten pH-Wert von 2 bis 13 verdickende Wirkung.

Die nach dem erfindungsgemäßen Verfahren erhältlichen Polyurethane haben weiterhin einen Einfluss auf die Struktur der Zubereitungen in dem sie die Feinteiligkeit der darin dispergierten Partikel vergrößern, d.h. die Teilchengröße erniedrigen.

Die nach dem erfindungsgemäßen Verfahren erhältlichen Polyurethane und deren Mischungen können auch dazu verwendet werden, Wasser enthaltende Zubereitungen herzustellen, die mindestens ein Salz oder mindestens ein Tensid oder Mischungen derselben enthalten.
Unter Tensiden werden im Zusammenhang mit der vorliegenden Erfindung auch Emulgatoren sowie Mischungen aus Tensiden und Emulgatoren verstanden. Unter Salz werden im Zusammenhang mit der vorliegenden Erfindung Salze und auch salzartige Strukturen auch mit niedrigem pKs Wert und Mischungen derselben verstanden

Besonders bevorzugt werden die erfindungsgemäß erhältlichen Polyurethane verwendet, um Zubereitungen herzustellen, die mindestens 0,05 Gew.-% Salz und/oder mindestens 0,5 Gew.-% Tenside enthalten, ganz besonders bevorzugt mindestens 0,1 % (w/w) Salz und/oder mindestens 1 Gew.-% Tenside enthalten.

In einer weiteren Ausführungsform werden die nach dem erfindungsgemäßen Verfahren erhältlichen Polyurethane dazu verwendet, Zubereitungen herzustellen, die mindestens 5 Gew.-%, bevorzugt mindestens 10 Gew.-% Salz enthalten.

In einer weiteren Ausführungsform werden die nach dem erfindungsgemäßen Verfahren erhältlichen Polyurethane verwendet, um Zubereitungen herzustellen, die bis 25 Gew.-% Tenside, bevorzugt bis 20 Gew.-% und besonders bevorzugt 15 Gew.-% oder weniger Tenside enthalten.

In einer weiteren Ausführungsform werden die nach dem erfindungsgemäßen Verfahren erhältlichen Polyurethane verwendet, um Zubereitungen herzustellen, die mindestens 1 Gew.-% Salz und bis 20 Gew.-% Tenside, bevorzugt bis 15 Gew.-% Tenside, enthalten.

Besonders bevorzugt werden die nach dem erfindungsgemäßen Verfahren erhältlichen Polyurethane dazu verwendet, Zubereitungen herzustellen, die Öl-in-Wasser-Emulsionen sind.

Typischerweise enthalten Öl-in-Wasser-Emulsionen Öl im Bereich von mehr als 0 bis 40 Gew.-%. Bevorzugt werden erfindungsgemäß Öl-in-Wasser-Emulsionen hergestellt, die einen Ölanteil im Bereich von 5 bis 40 Gew.-%, besonders im Bereich von 10 bis 35 Gew.-% und insbesondere von 15 bis 30 Gew.-% Öl enthalten.

Ganz besonders bevorzugt werden die nach dem erfindungsgemäßen Verfahren erhältlichen Polyurethane dazu verwendet, Zubereitungen herzustellen, die Öl-in-Wasser-Emulsionen sind und außerdem mindestens ein Salz enthalten.

Bei den erfindungsgemäßen Zubereitungen, die ein nach dem erfindungsgemäßen Verfahren erhältliches Polyurethan enthalten, kann es sich beispielsweise um Lösungen, Emulsionen, Suspensionen oder Dispersionen handeln.

In einer Ausführungsform ist eine erfindungsgemäße Zubereitung eine Dispersion, bevorzugt eine wässrige Dispersion, der nach dem erfindungsgemäßen Verfahren erhältlichen Polyurethane, wie sie nach dem Herstellungsprozess durch Aufarbeitung aus den Reaktionsprodukten erhalten werden kann. Dazu wird beispielsweise der Reaktionsmischung nach der Reaktion Wasser zugegeben und eine Dispersion erzeugt. Gewünschtenfalls kann auch die Zugabe eines Konservierungsmittels und/oder Stabilisators erfolgen.

In einer Ausführungsform enthält eine erfindungsgemäße Dispersion bis zu 50 Gew.-% der erfindungsgemäß erhältlichen Polyurethane.

In einer anderen Ausführungsform enthält eine erfindungsgemäße Dispersion 25 Gew.-% Feststoffanteil.

Ganz besonders bevorzugt sind wässrige Dispersionen enthaltend bis zu 25 Gew.-% der erfindungsgemäß erhältlichen Polyurethane, mindestens einen der zuvor beschriebenen, für kosmetische Anwendungen geeigneten Konservierungsstoffe und wahlweise mindestens einen der zuvor beschriebenen für kosmetische Anwendungen geeigneten Stabilisatoren.

In einer anderen Ausführungsform liegt das erfindungsgemäß erhältliche Polyurethan als Pulver vor. Ein solches Pulver kann beispielsweise durch Sprühtrocknung oder Gefriertrocknung der wässrigen Dispersion erhalten werden.

Zur Herstellung der erfindungsgemäßen Zubereitungen, die beispielsweise Lösungen, Emulsionen, Suspensionen oder Dispersionen sein können, werden die erfindungsgemäßen Polyurethane bevorzugt in Form von wässrigen Dispersionen oder als Pulver eingesetzt, wie sie beispielsweise aus dem Herstellungsprozess durch entsprechende Aufarbeitung erhalten werden können.

In den erfindungsgemäßen Zubereitungen können je nach geplanter Verwendung weitere Inhaltsstoffe enthalten sein.

Die die erfindungsgemäßen Polyurethane enthaltenden Zubereitungen können weitere Verdickungsmittel enthalten. Solche weiteren Verdickungsmittel sind dem Fachmann bekannt. Geeignete Verdicker sind beispielsweise in "Kosmetik und Hygiene von Kopf bis Fuß", Hrsg. W. Umbach, 3. Auflage, Wiley-VCH, 2004, S.235-236 genannt. Geeignete weitere Verdickungsmittel für die erfindungsgemäßen Zubereitungen sind beispielsweise auch auf Seite 37, Zeile 12 bis Seite 38, Zeile 8 der WO 2006/106140 beschrieben. Auf den Inhalt der genannten Textstellen wird hiermit in vollem Umfang Bezug genommen.

In einer Ausführungsform dieser Erfindung werden jedoch zur Herstellung der erfindungsgemäßen Zubereitungen neben den erfindungsgemäßen Polyurethanen keine weiteren Verdickungsmittel verwendet.

Bevorzugt sind erfindungsgemäße Polyurethane, deren 10 Gew.-%ige wässrige Dispersionen bei einer Schergeschwindigkeit von 100 1/s eine dynamische Viskosität (gemessen wie nachfolgend beschrieben) von mindestens 100 mPa*s, besonders bevorzugt von mindestens 200 mPa*s und ganz besonders bevorzugt von mindestens 300 mPa*s aufweisen.

Die wässrigen Dispersionen der nach dem erfindungsgemäßen Verfahren erhältlichen Polyurethane können dabei entweder newtonsches oder aber strukturviskoses Verhalten zeigen. Strukturviskose 10 Gew.-%ige wässrige Dispersionen, welche die nach dem erfindungsgemäßen Verfahren erhältlichen Polyurethane enthalten, weisen bei einer Schergeschwindigkeit von 100 1/s dynamische Viskositäten von mindestens 1000 mPa*s, besonders bevorzugt von mindestens 3000 mPa*s auf.

Dem Fachmann ist bekannt, dass in Wasser enthaltenden Zubereitungen viele Verdickungsmittel ihre Wirkung einbüssen, d.h. die Viskosität der Zubereitung sinkt, sobald die Zubereitungen Salze oder Tenside enthalten. Dahingegen ermöglichen die erfindungsgemäßen Polyurethane eine stabile Viskosität von wässrigen Zubereitungen auch bei Zugabe von Salzen und /oder Tensiden.

Besonders bevorzugt sind erfindungsgemäße Polyurethane, die bei einer Salzkonzentration von mindestens 0,5 Gew.-% nach Zugabe zu einer Stabilisierung der dynamischen Viskosität, gemessen wie nachfolgend beschrieben, der sie enthaltenden wässrigen Zubereitungen führen.

Insbesondere bevorzugt sind solche Polyurethane, die eine stabile dynamische Viskosität auch bei Zugabe von mindestens 0,5 Gew.-% Salz und Zugabe von mindestens 1 Gew.-% Tensid ermöglichen.

In einer weiteren Ausführungsform führt die Gegenwart der erfindungsgemäßen Polyurethane in salzhaltigen wässrigen Zubereitungen zur Erhöhung der Viskosität im Vergleich zu Zubereitungen, die nur Salz oder nur erfindungsgemäße Polyurethane enthalten.

Dabei spielt die Reihenfolge keine Rolle, in der Polyurethan und Salz zugegeben werden.

Besonders bevorzugt sind erfindungsgemäße Polyurethane, die zu einer Erhöhung der dynamischen Viskosität von wässrigen salz- und/oder tensidhaltigen Zubereitungen führen.

Insbesonders bevorzugt sind erfindungsgemäße Polyurethane, die bei einer Salzkonzentration von größer oder gleich 0,5 Gew.-% zu einer Erhöhung der dynamischen Viskosität der wässrigen Zubereitung führen.

Bevorzugt sind erfindungsgemäße Polyurethane, die bei einer Salzkonzentration der wässrigen Zubereitung von mindestens 0,05 Gew.-% zu einer Erhöhung der dynamischen Viskosität führen. Besonders bevorzugt sind solche Polyurethane, die zu einer Steigerung der dynamischen Viskosität im Vergleich zu Zubereitungen, die weniger als 0,05 Gew.-%, bevorzugt kleiner gleich 0,01 Gew.-%, Salz, oder weniger als 0,5 Gew.-%, bevorzugt kleiner gleich 0,1 Gew.-% Tensid enthalten, führen.

Ein weiterer Vorteil der nach dem erfindungsgemäßen Verfahren erhältlichen Polyurethane ist die Mizellenbildung in Wasser. Die kritische Stoffkonzentration zur Mizellenbildung, auch kritische Mizellenkonzentration (im Englischen "Critical Micelle Concentration" CMC) genannt, gibt die Konzentration eines Stoffes, meist eines Stoffes der hydrophobe und hydrophile Abschnitte innehat, an, bei der spontan Mizellen mit einer mittleren Teilchengröße kleiner gleich 200 nm, insbesondere kleiner gleich 100 nm (bestimmbar mittels dynamischer Lichtstreuung wie nachfolgend beschrieben) gebildet werden. Die CMC der erfindungsgemäßen Polyurethane in Wasser, ermittelt wie nachfolgend beschrieben, ist bevorzugt kleiner gleich 1 g/L, besonders bevorzugt kleiner gleich 0,5 g/L, insbesonders bevorzugt kleiner gleich 0,25 g/L und ganz besonders bevorzugt kleiner gleich 0,1 g/L.

Ein weiterer Vorteil des erfindungsgemäßen Verfahrens, der dadurch erhältlichen Polyurethane und der erfindungsgemäßen Zubereitungen ist die Verwendung von (Erd)alkalicarboxylat-Katalysatoren und damit der gleichzeitige Verzicht auf kosmetisch bedenkliche Katalysatoren bei der Herstellung der Polyurethane.

Im Bereich der kosmetischen Zubereitungen sind die bekannten Verfahren mit Zinn nicht länger erwünscht, da auch in den Produkten und daraus resultierenden Zubereitungen Zinn enthalten sein kann.

Die Verwendung der (Erd)alkalicarboxylate als Katalysatoren für das erfindungsgemäße Verfahren ermöglicht die in-situ-Erzeugung von Allophanat- und Isocyanurat-Strukturen und damit die ökonomisch vorteilhafte Erzeugung von verzweigten hydrophoben Abschnitten D der Polyurethane. Durch verzweigte Abschnitte D können Polyurethan-Verdicker mit höherer Effizienz erhalten werden.

Die erfindungsgemäßen Polyurethane können aufgrund ihrer Toleranz gegenüber hohen Salz- und gleichzeitig hohen Tensidgehalten auch bei extremen pH-Werten vorteilhaft auch in Home- Care-Zubereitungen wie beispielsweise Flüssigreinigungsmitteln als Verdicker verwendet werden.

Insbesondere sind die erfindungsgemäßen Polyurethane auch als Rheologiemodizierungsmittel für wasserstoffperoxidhaltige Zubereitungen geeignet.

### Kosmetische Zubereitungen

Die erfindungsgemäß erhältlichen Polyurethane werden bevorzugt in kosmetischen Zubereitungen verwendet. Ein Gegenstand der Erfindung sind also kosmetische Zubereitungen enthaltend die erfindungsgemäß erhältlichen Polyurethane.

Eine Ausführungsform der Erfindung sind Wasser enthaltende kosmetische Zubereitungen enthaltend erfindungsgemäß erhältliche Polyurethane.

Die erfindungsgemäßen Zubereitungen können als wässrige oder wässrig-alkoholische Lösungen, O/W- (bevorzugt) sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousses vorliegen und dementsprechend mit üblichen weiteren Hilfsstoffen formuliert werden.

Vorzugsweise liegen die erfindungsgemäßen Zubereitungen in Form eines Gels, Schaums, Mousse, Sprays, einer Salbe, Creme, Emulsion, Suspension, Lotion, Milch oder Paste vor.

Die Erfindung betrifft bevorzugt kosmetische Zubereitungen, die ausgewählt sind aus Gelen, Gelcremes, Milche, Hydroformulierungen, Stiftformulierungen, kosmetischen Ölen und Ölgelen, Mascara, Selbstbräunern, Gesichtspflegemitteln, Körperpflegemitteln, After-Sun-Präparaten. Unter dem Begriff kosmetische Zubereitungen werden auch Zubereitungen für die Mundpflege verstanden.

Weitere erfindungsgemäße kosmetische Zubereitungen sind hautkosmetische Zubereitungen, insbesondere solche zur Pflege der Haut. Diese liegen insbesondere als W/O- oder bevorzugt O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weitere bevorzugte erfindungsgemäße Zubereitungen sind Gesichtsmasken, kosmetische Lotionen und Zubereitungen für die Verwendung in der dekorativen Kosmetik, beispielsweise für Abdeckstifte, Theaterfarben, Mascara und Lidschatten, Lippenstifte, Kajalstifte, Eyeliner, Makeups, Grundierungen, Rouges, Puder und Augenbrauenstifte.

Weitere erfindungsgemäße Zubereitungen sind Antiaknemittel, Repellentien, Rasiermittel, Haarentfernungsmittel, Intimpflegemittel, Fußpflegemittel sowie Babypflegeprodukte.

Weitere bevorzugte erfindungsgemäße Zubereitungen sind Wasch-, Dusch- und Badepräparate. Wasch-, Dusch- und Badepräparaten sind im Rahmen dieser Erfindung Seifen von flüssiger bis gelförmiger Konsistenz, Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und - gele, Schaumbäder, Ölbäder und Scrub-Präparate, Rasierschäume, -lotionen und -cremes.

Kosmetische Zubereitungen, die spezielle Polyurethane enthalten, sind beispielsweise in WO 2009/135857 beschrieben. Die erfindungsgemäß erhältlichen Polyurethane der vorliegenden Erfindung sind generell auch für die Verwendung in den in WO 2009/135857 beschriebenen Zubereitungen geeignet. Auf die Offenbarung der WO 2009/135857 wird hiermit ausdrücklich Bezug genommen.

Im Rahmen der vorliegenden Erfindung werden die in den Zubereitungen der WO 2009/135857 verwendeten Polyurethane durch die Polyurethane dieser Erfindung ersetzt. Die erfindungsgemäßen Polyurethane werden also in den Zubereitungen der WO 2009/135857 bevorzugt an Stelle der dort verwendeten Polyurethane eingesetzt.

Geeignete Inhaltsstoffe für die erfindungsgemäßen Zubereitungen sind in WO 2009/135857, S.24 bis S.35 beschrieben, worauf in vollem Umfang Bezug genommen wird.

Erfindungsgemäß sind beispielsweise kosmetische UV-Lichtschutzmittel enthaltend die erfindungsgemäß erhältlichen Polyurethane. Unter kosmetischen Lichtschutzmittel werden im Rahmen dieser Erfindung kosmetische Zubereitungen verstanden, die wenigstens eine, bevorzugt mehrere UV-Filtersubstanzen enthalten.

Den erfindungsgemäßen UV- Lichtschutzmittel-Zubereitungen entsprechende UV-Lichtschutzmittel sind in WO 2009/135857, S.35 bis S.42 beschrieben, worauf in vollem Umfang Bezug genommen wird.

Die Erfindung betrifft auch kosmetische Zubereitungen, vorzugsweise in flüssiger oder pastöser Form, zur Verwendung auf der Haut, auf Semischleimhäuten, auf Schleimhäuten und insbesondere auf keratinischen Materialien wie Haaren, Wimpern und Augenbrauen, insbesondere zum Formen, Dekorieren, Färben, Verschönen derselben sowie zum Pflegen der Haut und der Hautanhangsgebilde. Grundsätzlich können die erfindungsgemäßen Zubereitungen bei geeigneter Einstellung und Einfärbung auch als Make-up, Concealer, Camouflage, Lidschatten, Eyeliner, Lipliner, Rouge, Lippenrouge, Lipgloss, Sonnenschutzmittel, Sunblocker, temporäres Tattoo, farbiger Effekt-Sonnenschutz für Surfer und dergleichen verwendet werden.

Eine bevorzugte Ausführungsform der vorliegenden Erfindung sind also kosmetische Zubereitungen für die dekorative Kosmetik.

Den erfindungsgemäßen Zubereitungen für die dekorative Kosmetik entsprechende Zubereitungen sind in WO 2009/135857, S.43 bis S.46 beschrieben, worauf in vollem Umfang Bezug genommen wird.

Ein Gegenstand der vorliegenden Erfindung sind wässrige Zubereitungen, die neben den erfindungsgemäß erhältlichen Polyurethanen weiterhin wenigstens ein Salz oder Tensid oder beides enthalten.

Eine weitere Ausführungsform der Erfindung sind Shampoos und kosmetische Reinigungsmittel enthaltend die erfindungsgemäß erhältlichen Polyurethane.

Den erfindungsgemäßen Shampoos und kosmetischen Reinigungsmitteln entsprechende Zubereitungen sind analog beschrieben in WO 2009/135857, S.46 bis S.55, worauf in vollem Umfang Bezug genommen wird.

Eine weitere Ausführungsform der Erfindung sind die erfindungsgemäß erhältlichen Polyurethane enthaltende Deodorantien oder Antitranspirantien, insbesondere Deo-Lotionen und Deodorant- oder Antitranspirant-Stifte, auf Basis von Öl-in-Wasser-Dispersion oder -Emulsion zur Applikation von Wirkstoffen, insbesondere von wasserlöslichen Wirkstoffen, auf die Haut.

Den erfindungsgemäßen Deodorantien und Antitranspirantien entsprechende Zubereitungen sind analog beschrieben in WO 2009/135857, S.55 bis S.59, worauf in vollem Umfang Bezug genommen wird.

Eine weitere Ausführungsform der Erfindung sind die erfindungsgemäß erhältlichen Polyurethane enthaltende Haarfärbemittel.

Den die erfindungsgemäß erhältlichen Polyurethane enthaltenden Haarfärbemitteln entsprechende Zubereitungen sind analog beschrieben in WO 2009/135857, S.59 bis S.65, worauf in vollem Umfang Bezug genommen wird.

Eine weitere Ausführungsform der Erfindung sind die erfindungsgemäß erhältlichen Polyurethane enthaltende Haarpflegemittel, insbesondere Haarkonditioniermittel.

Den die erfindungsgemäß erhältlichen Polyurethane enthaltenden Haarpflegemitteln entsprechende Haarpflegemittel sind analog beschrieben in WO 2009/135857, S.59 bis S.67, worauf in vollem Umfang Bezug genommen wird.

Eine weitere Ausführungsform der Erfindung sind die erfindungsgemäß erhältlichen Polyurethane enthaltende saure Zubereitungen.

Zahlreiche kosmetische Zubereitungen umfassen Wirkstoffe, die ihre gewünschte Wirkung insbesondere bei sauren pH-Werten entfalten. Dazu gehören beispielsweise Zubereitungen, die alpha-Hydroxycarbonsäuren (AHA) und beta-Hydroxycarbonsäuren (BHA) umfassen, da diese in neutralisiertem Zustand wenig bis nicht wirksam sind. Den die erfindungsgemäß erhältlichen Polyurethane enthaltenden sauren Zubereitungen entsprechende saure Zubereitungen sind analog beschrieben in WO 2009/135857, S.67 bis S.69, worauf in vollem Umfang Bezug genommen wird.

Eine weitere Ausführungsform der Erfindung sind die erfindungsgemäß erhältlichen Polyurethane enthaltende Selbstbräunungsprodukte.

Handelsübliche Selbstbräunungsprodukte stellen im allgemeinen O/W-Emulsionen dar. In diesen ist die Wasserphase durch in der Kosmetik gebräuchliche Emulgatoren stabilisiert.

Durch die Anwendung der erfindungsgemäßen Selbstbräunungsprodukte lässt sich nicht nur eine gleichmäßige Hautfärbung erreichen, es lassen sich auch von Natur aus oder durch krankhafte Veränderung unterschiedlich gefärbte Hautbereiche gleichmäßig einfärben.

Als selbstbräunende Substanzen werden erfindungsgemäß vorteilhaft unter anderem Glycerolaldehyd, Hydroxymethylglyoxal, γ-Dialdehyd, Erythrulose, 5-Hydroxy-1,4-naphtöchinon (Jug Ion) sowie das in den Henna-Blättern vorkommende 2-Hydroxy-I ,4-naphtochinon eingesetzt. Ganz besonders bevorzugt ist 1,3-Dihydroxyaceton (DHA), ein im menschlichen Körper vorkommender dreiwertiger Zucker. Auch 6-Aldo-D-Fructose und Ninhydrin können als erfindungsgemäße Selbstbräuner eingesetzt werden. Als Selbstbräuner im Sinne der Erfindung sind auch Substanzen zu verstehen, die eine von Braunton abweichende Hautfärbung hervorrufen.

In einer bevorzugten Ausführungsform der Erfindung enthalten solche Zubereitungen selbstbräunende Substanzen in einer Konzentration von 0,1 bis 10 Gew.-% und besonders bevorzugt von 0,5 bis 6 Gew.-%, jeweils bezogen auf das Gesamtgewicht der Zubereitung.

Bevorzugt enthalten diese Zubereitungen als selbstbräunende Substanz 1,3-Dihydroxyaceton. Weiter bevorzugt enthalten diese Zusammensetzungen organische und/oder anorganische Lichtschutzfilter. Die Zubereitungen können auch anorganische und/oder organische und/oder modifizierte anorganische Pigmente enthalten.

Weitere in den erfindungsgemäßen Zubereitungen bevorzugt enthaltene Inhaltsstoffe sind beispielsweise in der DE 103 21 147 in den Abschnitten [0024] bis [0132] genannt, worauf an dieser Stelle in vollem Umfang Bezug genommen wird.

Ein Gegenstand der Erfindung ist auch die Verwendung solcher Zubereitungen zur Färbung der Haut von mehrzelligen Organismen, insbesondere der Haut von Mensch und Tier, insbesondere auch zur Farbangleichung von unterschiedlich pigmentierten Hautstellen.

Eine weitere Ausführungsform der Erfindung sind die erfindungsgemäß erhältlichen Polyurethane enthaltende Zubereitungen zur Mund- und Zahnpflege- und -reinigung.

Den die erfindungsgemäß erhältlichen Polyurethane enthaltenden Zubereitungen zur Mund- und Zahnpflege- und -reinigung entsprechende Zubereitungen sind analog beschrieben in WO 2009/135857, S.69 bis S.70, worauf in vollem Umfang Bezug genommen wird.

Eine weitere Ausführungsform der Erfindung sind die erfindungsgemäß erhältlichen Polyurethane enthaltende Zubereitungen für die Haarentfemung.

Den die erfindungsgemäß erhältlichen Polyurethane enthaltenden Zubereitungen für die Haarentfernung entsprechende Zubereitungen sind analog beschrieben in WO 2009/135857, S.70 bis S.71, worauf in vollem Umfang Bezug genommen wird.

Eine weitere Ausführungsform der Erfindung sind die erfindungsgemäß erhältlichen Polyurethane enthaltende Zubereitungen für die dauerhafte Haarverformung.

Den die erfindungsgemäß erhältlichen Polyurethane enthaltenden Zubereitungen für die dauerhafte Haarverformung entsprechende Zubereitungen sind analog beschrieben in WO 2009/135857, S.71 bis S.73, worauf in vollem Umfang Bezug genommen wird.

### Beispiele

Die Erfindung wird anhand der folgenden nicht einschränkenden Beispiele näher erläutert.

Soweit nichts anderes angegeben ist, sind die Prozentangaben Gewichtsprozent.

### Bestimmung der dynamischen Viskosität

Die dynamischen Viskositäten der erfindungsgemäßen Polyurethane wurden jeweils in einer 10 Gew.-%igen wässrigen Lösung bei 23°C gemessen. In den unten aufgeführten Beispielen wurde dazu stets die dynamische Viskosität bei Schergeschwindigkeiten von 100 1/s und 350 1/s bestimmt. Diese beiden Werte erlauben eine Aussage darüber, ob die erfindungsgemäßen Polyurethane in wässriger Dispersion strukturviskoses oder newtonsches Verdickerverhalten zeigen.

Zur Bestimmung der dynamischen Viskosität nach DIN 53019 wurden verwendet:
- Rotationsviskosimeter Physica Rheolab MCI Portable (Firma Anton Paar);
- Zylinder Messsystem, Z4 DIN Zylinder (Durchmesser 14 mm)

Die im Folgenden verwendeten Polyole, ethoxylierten Alkohole und Isocyanate wurden in im Wesentlichen alkalifreier Form eingesetzt (Kaliumgehalt <5 ppm).

### Synthesebeispiel 1: Herstellung eines PUR-Assoziativverdickers A.1

### Katalysator: 300 ppm K als Kaliumacetat

120,00 g Polyethylenglykol Pluriol^{®}E6000 (BASF SE, Molekulargewicht 6000 g/mol), 9,60 g Lutensol^{®}T010 (BASF SE), 11,10 g Lutensol^{®}AT11 (BASF SE), und 106 mg Kaliumacetat (= 300 ppm Kalium bezogen auf Gesamtansatz) wurden unter Stickstoff in einem 2-Liter-Polymerisationsreaktor vorgelegt und anschließend auf 100 °C erhitzt. Durch Anlegen eines Vakuums von ca. 10 mbar für 6 Stunden wurden Wasserspuren soweit entfernt, bis schließlich der Wassergehalt des Ansatzes 230 ppm betrug. Nun wurde das Gemisch auf 80 °C abgekühlt. Durch Zugabe von 5,88 g Hexamethylendiisocyanat wurde die Polymerisation gestartet und der Ansatz bei einer Temperatur von 80 °C für 50 Minuten gerührt, bis ein Isocyanat-Gehalt von 0,0 % erreicht war. Nun wurde der gelb gefärbte Rückstand in 586,3 g Wasser aufgelöst und die wässrige Lösung umgehend mit 7,33 g Euxyl^{®}K701 (Schülke&Mayr) sowie 70 mg des Stabilisators 4-Hydroxy-TEMPO versetzt. Das Gemisch wurde auf Raumtemperatur (25 °C) abgekühlt. Das Polymer A.1 (Mn = 11700 g/mol; M_{w} = 26900 g/mol) wurde so in Form einer klaren, schwach gelb gefärbten Lösung erhalten, welche einen Feststoffgehalt von 20,8 % aufwies. Die Viskosität einer 10 Gew.-%igen wässrigen Lösung des Polyetherpolyurethans A.1 betrug 1550 mPa*s (Scherrate 100 1/s) bzw. 1360 mPa*s (Scherrate 350 1/s).

### Synthesebeispiel 2: Herstellung eines PUR-Assoziativverdickers A.2

### Katalysator: 600 ppm K als Kaliumacetat

120,00 g Polyethylenglykol Pluriol^{®}E6000, 9,60 g Lutensol^{®}T010, 11,10 g Lutensol^{®}AT11, und 212 mg Kaliumacetat (= 600 ppm Kalium bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 5 Stunden wurden Wasserspuren soweit entfernt, bis schließlich der Wassergehalt des Ansatzes 225 ppm betrug. Nun wurde das Gemisch auf 80 °C abgekühlt. Durch Zugabe von 5,88 g Hexamethylendiisocyanat wurde die Polymerisation gestartet und der Ansatz bei einer Temperatur von 80 °C für 50 Minuten gerührt; bis ein Isocyanat-Gehalt von 0,0 % erreicht war. Nun wurde der gelb gefärbte Rückstand in 586,3 g Wasser aufgelöst und die wässrige Lösung umgehend mit 7,33 g Euxyl^{®}K701 sowie 70 mg des Stabilisators 4-Hydroxy-TEMPO versetzt. Das Gemisch wurde auf Raumtemperatur (25 °C) abgekühlt. Das Polymer A.2 (Mₙ = 12300 g/mol; M_{w} = 29700 g/mol) wurde so in Form einer klaren, schwach gelb gefärbten Lösung erhalten, welche einen Feststoffgehalt von 20,1 % aufwies. Die Viskosität einer Gew.-10 %igen wässrigen Lösung des Polyetherpolyurethans A.2 betrug 3700 mPa*s (Scherrate 100 1/s) bzw. 3000 mPa*s (Scherrate 350 1/s).

### Synthesebeispiel 3: Herstellung eines PUR-Assoziativverdickers A.3

### Katalysator: 900 ppm K als Kaliumacetat

120,00 g Polyethylenglykol Pluriol^{®}E6000, 9,60 g Lutensol^{®}TO10, 11,10 g Lutensol^{®}AT11, und 318 mg Kaliumacetat (= 900 ppm Kalium bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 4 Stunden wurden Wasserspuren soweit entfernt, bis schließlich der Wassergehalt des Ansatzes 242 ppm betrug. Nun wurde das Gemisch auf 80 °C abgekühlt. Durch Zugabe von 5,88 g Hexamethylendiisocyanat wurde die Polymerisation gestartet und der Ansatz bei einer Temperatur von 80 °C für 55 Minuten gerührt, bis ein Isocyanat-Gehalt von 0,0 % erreicht war. Nun wurde der gelb gefärbte Rückstand in 586,3 g Wasser aufgelöst und die wässrige Lösung umgehend mit 7,33 g des Konservierungsmittels Euxyt^{®}K701 sowie 70 mg des Stabilisators 4-Hydroxy-TEMPO versetzt. Das Gemisch wurde auf Raumtemperatur (25 °C) abgekühlt. Das Polymer A.3 (Mₙ = 12700 g/mol; M_{w} = 33000 g/mol) wurde so in Form einer klaren, schwach gelb gefärbten Lösung erhalten, welche einen Feststoffgehalt von 20,6 % aufwies. Die Viskosität einer 10 Gew.-%igen wässrigen Lösung des Polyetherpolyurethans A.3 betrug 6000 mPa*s (Scherrate 100 1/s) bzw. 4400 mPa*s (Scherrate 350 1/s).

### Synthesebeispiel 4: Herstellung eines PUR-Assoziativverdickers A.4

### Katalysator: 2000 ppm K als Kaliumacetat

90,00 g Polyethylenglykol Pluriol^{®}E6000, 7,20 g Lutensol^{®}TO10, 8,33 g Lutensol^{®}AT11, und 530 mg Kaliumacetat (= 2000 ppm Kalium bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 5 Stunden wurden Wasserspuren soweit entfernt, bis schließlich der Wassergehalt des Ansatzes 210 ppm betrug. Nun wurde das Gemisch auf 80 °C abgekühlt. Durch Zugabe von 4,41 g Hexamethylendiisocyanat wurde die Polymerisation gestartet und der Ansatz bei einer Temperatur von 80 °C für 55 Minuten gerührt, bis ein Isocyanat-Gehalt von 0,0 % erreicht war. Nun wurde der gelb gefärbte Rückstand in 439,7 g Wasser aufgelöst und die wässrige Lösung umgehend mit 5,50 g des Konservierungsmittels Euxyl^{®}K701 sowie 50 mg des Stabilisators 4-Hydroxy-TEMPO versetzt. Das Gemisch wurde auf Raumtemperatur (25 °C) abgekühlt. Das Polymer A.4 (Mₙ = 12500 g/mol; M_{w} = 31300 g/mol) wurde so in Form einer klaren, schwach gelb gefärbten Lösung erhalten, welche einen Feststoffgehalt von 20,6 % aufwies. Die Viskosität einer 10 Gew.-%igen wässrigen Lösung des Polyetherpolyurethans A.4 betrug 5200 mPa*s (Scherrate 100 1/s) bzw. 3800 mPa*s (Scherrate 350 1/s).

### Synthesebeispiel 5: Herstellung eines PUR-Assoziativverdickers A.5

### Katalysator: 200 ppm K als Kaliumacetat

100,00 g Polyethylenglykol (Merck KGaA, Molekulargewicht 10000 g/mol), 10,02 g 2-Decyl-1-tetradecanol-20 EO, und 55 mg Kaliumacetat (= 200 ppm Kalium bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 6 Stunden wurden Wasserspuren soweit entfernt, bis schließlich der Wassergehalt des Ansatzes 190 ppm betrug. Nun wurde das Gemisch auf 80°C abgekühlt. Durch Zugabe von 2,52 g Hexamethylendiisocyanat wurde die Polymerisation gestartet und der Ansatz bei einer Temperatur von 80°C für 40 Minuten gerührt, bis ein Isocyanat-Gehalt von 0,0 % erreicht war. Nun wurde der gelb gefärbte Rückstand in 450,2 g Wasser aufgelöst und die wässrige Lösung umgehend mit 5,63 g des Konservierungsmittels Euxyl^{®}K701 sowie 60 mg des Stabilisators 4-Hydroxy-TEMPO versetzt. Das Gemisch wurde auf Raumtemperatur (25 °C) abgekühlt. Das Polymer A.5 (Mₙ = 19100 g/mol; M_{w} = 48500 g/mol) wurde so in Form einer klaren, schwach gelb gefärbten Lösung erhalten, welche einen Feststoffgehalt von 20,7 % aufwies. Die Viskosität einer 5 Gew.-%igen wässrigen Lösung des Polyetherpolyurethans A.5 betrug >20000 mPa*s (Scherrate 100 1/s) bzw. >20000 mPa*s (Scherrate 350 1/s).

### Vergleichsbeispiel 1: Herstellung eines PUR-Assoziativverdickers A.6

### ohne Katalysator

120,00 g Polyethylenglykol Pluriol^{®}E6000, 9,60 g Lutensol^{®}TO10, und 11,10 g Lutensol^{®}AT11, wurden unter Stickstoff in einem 2-L-Polymerisationsreaktor vorgelegt und anschließend auf 100 °C erhitzt. Durch Anlegen eines Vakuums von ca. 10 mbar für 5 Stunden wurden Wasserspuren soweit entfernt, bis schließlich der Wassergehalt des Ansatzes 250 ppm betrug. Nun wurde das Gemisch auf 80 °C abgekühlt. Durch Zugabe von 5,88 g Hexamethylendiisocyanat wurde die Polymerisation gestartet und der Ansatz bei einer Temperatur von 80 °C für insgesamt 32 Stunden gerührt, bis ein Isocyanat-Gehalt von 0,0 % erreicht war. Nun wurde der gelb gefärbte Rückstand in 586,3 g Wasser aufgelöst und die wässrige Lösung umgehend mit 7,33 g des Konservierungsmittels Euxyl^{®}K701 sowie 70 mg des Stabilisators 4-Hydroxy-TEMPO versetzt. Das Gemisch wurde auf Raumtemperatur (25 °C) abgekühlt. Das Polymer A.6 (Mₙ = 10000 g/mol; M_{w} = 23200 g/mol) wurde so in Form einer klaren, schwach gelb gefärbten Lösung erhalten, welche einen Feststoffgehalt von 18,8 % aufwies. Die Viskosität einer 10 Gew.-%igen wässrigen Lösung des Polyetherpolyurethans A.6 betrug 100 mPa*s (Scherrate 100 1/s) bzw. 100 mPa*s (Scherrate 350 1/s).

### Vergleichsbeispiel 2: Herstellung eines PUR-Assoziativverdickers A.7

### ohne Katalysator

100,00 g Polyethylenglykol (Merck KGaA, Molekulargewicht 10000 g/mol), und 10,02 g 2-Decyl-1-tetradecanol-20 EO, wurden unter Stickstoff in einem 2-L-Polymerisationsreaktor vorgelegt und anschließend auf 100 °C erhitzt. Durch Anlegen eines Vakuums von ca. 10 mbar für 6 Stunden wurden Wasserspuren soweit entfernt, bis schließlich der Wassergehalt des Ansatzes 185 ppm betrug. Nun wurde das Gemisch auf 80 °C abgekühlt. Durch Zugabe von 2,52 g Hexamethylendiisocyanat wurde die Polymerisation gestartet und der Ansatz bei einer Temperatur von 80 °C für insgesamt 12 Stunden gerührt, bis ein Isocyanat-Gehalt von 0,0 % erreicht war. Nun wurde der gelb gefärbte Rückstand in 450,2 g Wasser aufgelöst und die wässrige Lösung umgehend mit 5,63 g des Konservierungsmittels Euxyl^{®}K701 sowie 60 mg des Stabilisators 4-Hydroxy-TEMPO versetzt. Das Gemisch wurde auf Raumtemperatur (25 °C) abgekühlt. Das Polymer A.7 (Mₙ = 5200 g/mol; M_{w} = 14500 g/mol) wurde so in Form einer klaren, schwach gelb gefärbten Lösung erhalten, welche einen Feststoffgehalt von 18,5 % aufwies. Die Viskosität einer 10 Gew.-%igen wässrigen Lösung des Polyetherpolyurethans A.7 betrug <100 mPa*s (Scherrate 100 1/s) bzw. <100 mPa*s (Scherrate 350 1/s).

### Vergleichsbeispiel 3: Herstellung eines PUR-Assoziativverdickers A.8

### Katalysator: Dibutylzinndilaurat

90,00 g Polyethylenglykol Pluriol^{®}E6000, 7,20 g Lutensol^{®}TO10, 8,33 g Lutensol^{®}AT11, und 144 mg Dibutylzinndilaurat (DBTL) (= 300 ppm Sn bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 5 Stunden wurden Wasserspuren soweit entfernt, bis schließlich der Wassergehalt des Ansatzes 250 ppm betrug. Nun wurde das Gemisch auf 80 °C abgekühlt. Durch Zugabe von 4,41 g Hexamethylendiisocyanat wurde die Polymerisation gestartet und der Ansatz bei einer Temperatur von 80 °C für 60 Minuten gerührt, bis ein Isocyanat-Gehalt von 0,0 % erreicht war. Nun wurde der gelb gefärbte Rückstand in 440,3 g Wasser aufgelöst und die wässrige Lösung umgehend mit 5,50 g des Konservierungsmittels Euxyl^{®}K701 sowie 60 mg des Stabilisators 4-Hydroxy-TEMPO versetzt. Das Gemisch wurde auf Raumtemperatur (25 °C) abgekühlt. Das Polymer A.8 (Mₙ = 10100 g/mol; M_{w} = 22000 g/mol) wurde so in Form einer klaren, schwach gelb gefärbten Lösung erhalten, welche einen Feststoffgehalt von 20,4 % aufwies. Die Viskosität einer 10 Gew.-%igen wässrigen Lösung des Polyetherpolyurethans A.8 betrug 720 mPa*s (Scherrate 100 1/s) bzw. 660 mPa*s (Scherrate 350 1/s).

### Vergleichsbeispiel 4: Herstellung eines PUR-Assoziativverdickers A.9

### Katalysator: DABCO

120,00 g Polyethylenglykol Pluriol^{®}E6000, 9,60 g Lutensol^{®}TO10, 11,10 g Lutensol^{®}AT11, und 36 mg 1,4-Diazabicyclo[2.2.2]octan (DABCO; = 300 ppm bezogen auf Gesamtansatz) wurden unter Stickstoff in einem 2-L-Polymerisationsreaktor vorgelegt und anschließend auf 100 °C erhitzt. Durch Anlegen eines Vakuums von ca. 10 mbar für 5 Stunden wurden Wasserspuren soweit entfernt, bis schließlich der Wassergehalt des Ansatzes 230 ppm betrug. Nun wurde das Gemisch auf 80 °C abgekühlt. Durch Zugabe von 5,88 g Hexamethylendiisocyanat wurde die Polymerisation gestartet und der Ansatz bei einer Temperatur von 80 °C für insgesamt 13 Stunden gerührt, bis ein Isocyanat-Gehalt von 0,0 % erreicht war. Nun wurde der gelb gefärbte Rückstand in 586,5 g Wasser aufgelöst und die wässrige Lösung umgehend mit 7,33 g des Konservierungsmittels Euxyl^{®}K701 sowie 70 mg des Stabilisators 4-Hydroxy-TEMPO versetzt. Das Gemisch wurde auf Raumtemperatur (25 °C) abgekühlt. Das Polymer A.9 (Mₙ = 8500 g/mol; M_{w} = 17400 g/mol) wurde so in Form einer klaren, schwach gelb gefärbten Lösung erhalten, welche einen Feststoffgehalt von 18,7 % aufwies. Die Viskosität einer 10 Gew.-%igen wässrigen Lösung des Polyetherpolyurethans A.9 betrug 105 mPa*s (Scherrate 100 1/s) bzw. 105 mPa*s (Scherrate 350 1/s).

### Formulierungsbeispiel 1:

### Kosmetische Formulierungen auf einer Cremophor® A6/ Cremophor® A25-Basis

Die im Folgenden gezeigten kosmetischen Formulierungen wurden durch Zugabe der Wasserphase B zur Ölphase A und anschließendem Versetzen der erhaltenen O/W-Emulsion mit dem Konservierungsmittel (Phase C) hergestellt. Man erhielt die folgenden auf einer Cremophor^{®} A6/ Cremophor^{®}A25-Grundlage basierenden Formulierungen.

**Tabelle 1.**

| Formulierungen auf Basis von Cremophor^{®} A6/ Cremophor^{®} A25 | | | | | | | |
|---|---|---|---|---|---|---|---|
| Phase | Inhaltsstoffe | F.1.1 | F.1.2 | F.1.3 | F.1.4 | F.1.6 | F.1.8 |
| Phase A | Cremophor^{®}A 6 | 2,0 g | 2,0 g | 2,0 g | 2,0 g | 2,0 g | 2,0 g |
| | Cremophor^{®}A 25 | 2,0 g | 2,0 g | 2,0 g | 2,0 g | 2,0 g | 2,0 g |
| | Lanette^{®}O | 2,5 g | 2,5 g | 2,5 g | 2,5 g | 2,5 g | 2,5 g |
| | Paraffinöl | 5,0 g | 5,0 g | 5,0 g | 5,0 g | 5,0 g | 5,0 g |
| | Luvitol^{®}EHO | 5,0 g | 5,0 g | 5,0 g | 5,0 g | 5,0 g | 5,0 g |
| Phase B | PUR-Verdicker | A.1 | A.2 | A.3 | A.4 | A.6 | A.8 |
| | | 0,5 g | 0,5 g | 0,5 g | 0,5 g | 0,5 g | 0,5 g |
| | 1,2-Propylenglykol | 5,0 g | 5,0 g | 5,0 g | 5,0 g | 5,0 g | 5,0 g |
| | Wasser | 77,5 g | 77,5 g | 77,5 g | 77,5 g | 77,5 g | 77,5 g |
| Phase C | Euxyl^{®}K300 | 0,5 g | 0,5 g | 0,5 g | 0,5 g | 0,5 g | 0,5 g |

### Formulierungsbeispiel 2:

### Kosmetische Formulierungen auf Stearatester-Basis

Die folgenden kosmetischen Formulierungen wurden durch Zugabe der Wasserphase B zur Ölphase A und anschließendem Versetzen der erhaltenen O/W-Emulsion mit dem Konservierungsmittel (Phase C) hergestellt. Man erhielt die folgenden auf einer Stearatester-Grundlage basierenden Formulierungen.

**Tabelle 2.**

| Kosmetische Formulierungen auf Basis einer Stearatester-Grundlage | | | | | | | |
|---|---|---|---|---|---|---|---|
| Phase | Inhaltsstoffe | F.2.1 | F.2.2 | F.2.3 | F.2.4 | F.2.6 | F.2.8 |
| Phase A | Cutina^{®}GMS | 2,0 g | 2,0 g | 2,0 g | 2,0 g | 2,0 g | 2,0 g |
| | Lanette^{®}18 | 2,0 g | 2,0 g | 2,0 g | 2,0 g | 2,0 g | 2,0 g |
| | Dow Corning 345 Fluid | 3,0 g | 3,0 g | 3,0 g | 3,0 g | 3,0 g | 3,0 g |
| | Cetiol^{®}OE | 3,0 g | 3,0 g | 3,0 g | 3,0 g | 3,0 g | 3,0 g |
| | Abil^{®}350 | 2,0 g | 2,0 g | 2,0 g | 2,0 g | 2,0 g | 2,0 g |
| | Dry Flo PC | 1,0 g | 1,0 g | 1,0 g | 1,0 g | 1,0 g | 1,0 g |
| | Myrj 52 | 2,0 g | 2,0 g | 2,0 g | 2,0 g | 2,0 g | 2,0 g |
| Phase B | PUR-Verdicker | A.1 | A.2 | A.3 | A.4 | A.6 | A.8 |
| | | 0,5 g | 0,5 g | 0,5 g | 0,5 g | 0,5 g | 0,5 g |
| | Glycerin | 5,0 g | 5,0 g | 5,0 g | 5,0 g | 5,0 g | 5,0 g |
| | Wasser | 79,0 g | 79,0 g | 79,0 g | 79,0 g | 79,0 g | 79,0 g |
| Phase C | Euxy®K300 | 0,5 g | 0,5 g | 0,5 g | 0,5 g | 0,5 g | 0,5 g |

### Viskositäten der kosmetischen Formulierungen F.1.1 bis F.1.8. in Abhängigkeit von der Salzkonzentration

**Tabelle 3.**

| Formulierung | Viskosität [mPa*s] mit 2,0 % NaCl Zusatz |
|---|---|
| F.1.1 | 12100 |
| F.1.2 | 17100 |
| F.1.3 | 26200 |
| F.1.4 | 17000 |
| | |
| F.1.6 | 6800 |
| F.1.8 | 11400 |

### Viskositäten der kosmetischen Formulierungen F.2.1 - F.2.8 in Abhängigkeit von der Salzkonzentration

**Tabelle 4.**

| Formulierung | Viskosität [mPa*s] mit 2,0 % NaCl Zusatz |
|---|---|
| F.2.1 | 16700 |
| F.2.2 | 21800 |
| F.2.3 | 21200 |
| F.2.4 | 18100 |
| | |
| F.2.6 | 10000 |
| F.2.8 | 14700 |

### Viskositäten der Polyurethane A.1 - A.9 in Wasser, in Abhängigkeit von Scherrate und Konzentration

**Tabelle 5.**

| Polymer | Polymerkonzentration in Wasser | Viskosität | |
|---|---|---|---|
| | | [mPa*s] | |
| | [Gew%] | Scherrate 100 1/s | Scherrate 350 1/s |
| A.1 | 10 | 1550 | 1360 |
| A.2 | 10 | 3700 | 3000 |
| A.3 | 10 | 6000 | 4400 |
| A.4 | 10 | 5200 | 3800 |
| A.5 | 5 | >20000 | >20000 |
| | | | |
| A.6 | 10 | 100 | 100 |
| A.7 | 10 | <100 | <100 |
| A.8 | 10 | 720 | 660 |
| A.9 | 10 | 105 | 105 |

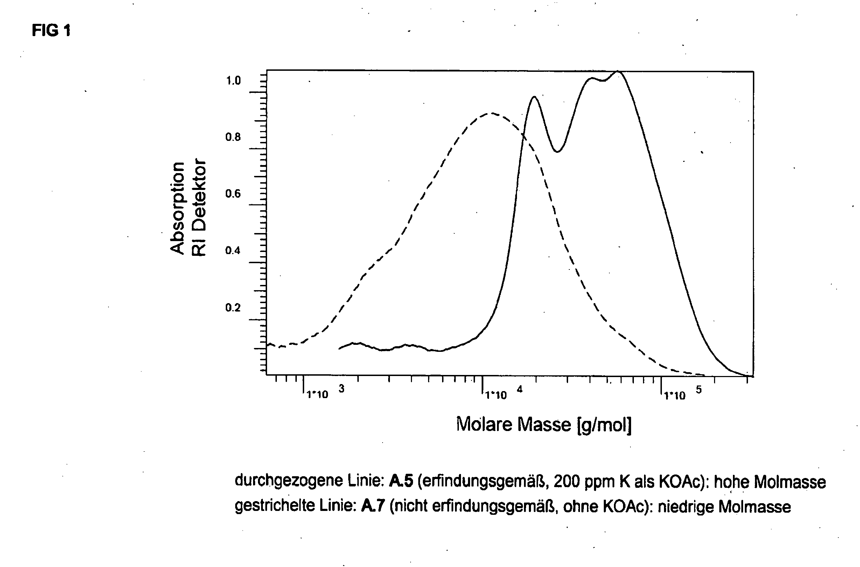

### Abbildung 1. GPC Chromatogramme der Polyurethane A.5 und A.7.

### Anwendungsbeispiele:

An dieser Stelle wird in vollem Umfang bezug genommen auf die anwendungstechnischen Beispiele, die in der WO 2009/135857 auf den Seiten 87 bis 114 offenbart sind.

Die in den dortigen Beispielen verwendeten Polyurethane PU.1 bis PU.11 werden für die Zwecke dieser Erfindung durch die Polyurethane A.1, A.2, A.3, A.4 oder A.5 der vorliegenden Erfindung ersetzt, so dass die entsprechenden kosmetischen Zubereitungen analog erhalten werden.

Im Folgenden sind weitere typische erfindungsgemäße Zubereitungen beschrieben ohne jedoch die Erfindung auf diese Beispiele zu beschränken.

Neben der hier beschriebenen Herstellung der kosmetischen Zubereitungen können die Polyurethane A.1, A.2, A.3, A.4 oder A.5 sowie Kombinationen daraus der entstandenen Emulsion auch nach Vereinigung von Wasser- und Ölphase bei 60-80°C oder der abgekühlten Emulsion bei etwa 40°C zugegeben werden.

Gegenstand der Erfindung ist auch die nachträgliche Zugabe der erfindungsgemäß erhältlichen Polyurethane zu einer kosmetischen Zubereitung, um die gewünschte Viskosität einzustellen.

Die Prozentangaben sind Gew.-%, sofern nicht ausdrücklich anderweitig beschrieben.

### O/W-Emulsion

| Phase | Inhaltsstoff/ INCI | F.3.1 | F.3.2 | F.3.3 | F.3.4 | F.3.5 |
|---|---|---|---|---|---|---|
| A | Aqua | ad 100 | ad 100 | ad 100 | ad 100 | ad 100 |
| | Glycerin | 3,0 | 5, 50 | 4,50 | 5,00 | 3,5 |
| | PUR-Verdicker A.1 | 3,0 | 1,5 | 0,8 | 2,0 | 2,5 |
| | Hydroxyethyl Acrylate/ Sodium Acryloyldimethyl Taurate Copolymer, Squalane, Polysorbate 60 | | 1,0 | | 0,5 | |
| B | Glyceryl Stearate Citrate | 1,80 | 2,00 | 3,00 | 1,50 | 2 |
| | Sucrose Stearate | 1,00 | 1,20 | 2,00 | 2,20 | 1,5 |
| | Cetearyl Alcohol | 1,80 | 2,00 | 1,50 | 2,40 | 2,8 |
| | Ethylhexyl Palmitate | 6,00 | 5,00 | 3,50 | 3,00 | 5,5 |
| | Caprylic/Capric Triglyceride | 5,00 | 5,00 | 1,00 | 2,00 | 3,5 |
| | Octyldodecanol | 1,50 | 3,00 | 2,40 | 5,0 | 4,6 |
| | Dimethicone | 0,20 | 0,50 | 2,00 | 1,80 | 1,4 |
| C | Ammonium Acryloyldimethyltaurate/ VP Copolymer | | | 0,5 | 0,1 | |
| | Carbomer | 0,05 | | | | 0,1 |
| D | Sodium Hydroxide | 0,02 | | | | 0,04 |
| E | Bisabolol | 0,5 | 0,3 | 0,20 | 0,35 | 1,0 |
| | Phenoxyethanol, Parabenmischung | 1,00 | 0,60 | 0,70 | 0,60 | 0,5 |
| | Parfum | 0.05 | 0,10 | 0,10 | 0,05 | 0,05 |

### Herstellung:

Die Phasen A und B getrennt auf ca. 80°C erwärmen. Phase C in Phase B einrühren und anschließend Phase A in Phase B/C einrühren und kurz homogenisieren.

Phase D (wenn benötigt) hinzugeben und unter Rühren auf ca. 40°C abkühlen. Komponenten der Phase E nacheinander zur Emulsion geben und unter Rühren auf Raumtemperatur abkühlen. Kurz homogenisieren.

Anstelle der O/W-Emulsion enthaltend Polyurethan-Verdicker A.1 werden auch O/W-Emulsionen enthaltend eines oder mehrere der Polyurethane A.2, A.3, A.4 oder A.5 hergestellt.

### Hydrodispersion

| Phase | Inhaltsstoff/ INCI | F.4.1 | F.4.2 | F.4.3 | F.4.4 | F.4.5 |
|---|---|---|---|---|---|---|
| A | Stearyl Alcohol | 0,5 | 1,5 | | | 2,0 |
| | Cetyl Alkohol | | | 1,00 | 2,5 | |
| | C12-15 Alkyl Benzoat | | 2,5 | | 4,0 | |
| | Dicapryl Ether | | 4,0 | | | 6,0 |
| | Butylenglycol Dicaprylat/Dicaprat | 4,0 | | 2,0 | 1,0 | |
| | Dicapryl Carbonat | | 2,0 | 3,0 | | 4,0 |
| | Cyclopentasiloxane, Cyclohexasiloxane | | | | 2,0 | 0,5 |
| | Prunus Amygdalus Dulcis (Sweet Almond) Oil | 2,0 | | 0,5 | | |
| | Shea Butter | | 2,0 | | 1,0 | |
| | Hydrogenated Polyisobutene | 3,0 | 1,0 | 7,0 | 0,5 | 2,0 |
| | Squalane | | | | 2,0 | 0,5 |
| | Vitamin E Acetate | 0,50 | | 0,25 | | 1,00 |
| B | Acrylat/C10-30 Alkyl Acrylat Crosspolymer | 0,3 | 0,1 | 0,2 | 0,15 | 0,2 |
| C | Aqua | ad 100 | ad 100 | ad 100 | ad 100 | ad 100 |
| | Polyacrylamide, C13-14 Isoparaffin, Laureth-7 | | 1,0 | 1,5 | 0,75 | |
| | PUR-Verdicker A.1 | 2,5 | 2,0 | 0,9 | 1,5 | 3,0 |
| | Propylene Glycol | 3,00 | 5,0 | 2,5 | 7,50 | 10,0 |
| D | Sodium Hydroxide | 0,12 | 0,04 | 0,08 | 0,06 | 0,08 |
| E | Niacinamide | 0,30 | 3,0 | 1,5 | 0,5 | 0,20 |
| | Aqua | 2,0 | 10,0 | 5,0 | 2,0 | 2,0 |
| F | DMDM Hydantoin | | 0,60 | 0,45 | 0,25 | |
| | Methylparaben | 0,50 | | 0,25 | 0,15 | |
| | Phenoxyethanol | 0,50 | 0,40 | | 1,00 | |
| | Ethylhexylglycerin | | | 1,00 | | 0,80 |
| | Ethanol | 3,00 | 2,00 | 1,50 | | 7,00 |
| G | Fragrance | 0,20 | | 0,05 | | 0,40 |

### Herstellung:

Die Phasen A und C getrennt auf ca. 80°C erwärmen.

Phase B in Phase A einrühren und anschließend Phase C in Phase A/B. Kurz homogenisieren. Phase D hinzugeben und unter Rühren auf ca. 40°C abkühlen. Phase E zugeben und unter Rühren auf ca. 30°C abkühlen. Phase F und G zur Emulsion geben und unter Rühren auf Raumtemperatur abkühlen. Kurz homogenisieren.

Anstelle der Hydrodispersion enthaltend Polyurethan-Verdicker A.1 werden auch Hydrodispersionen enthaltend eines oder mehrere der Polyurethane A.2, A.3, A.4 oder A.5 hergestellt.

### Feststoffstabilisierte Emulsion

| Phase | Inhaltsstoff/ INCI | F.5.1 | F.5.2 | F.5.3 | F.5.4 | F.5.5 |
|---|---|---|---|---|---|---|
| A | Mineral Oil | 4,0 | 6,0 | 16,0 | 10,0 | 6,0 |
| | Octyldodecanol | 9,0 | 9,0 | 5,0 | | |
| | Ethylhexyl Isononanoate | 9,0 | 9,0 | 6,0 | 5,0 | 8,0 |
| | Isohexadecane | 9,0 | 5,0 | | 4,0 | 8,0 |
| | Dimethicone | 0,5 | 2,0 | 1,0 | | 1,5 |
| | Cera Microcristallina, Paraffinum Liquidum | | 0,35 | | 0,75 | 3,0 |
| | Phenyl trimethicone | 2,0 | | 1,0 | 2,5 | 3,0 |
| | Silica | 2,5 | | | 6,0 | 2,5 |
| | Aluminum Starch Octenylsuccinate | 2,0 | 1,0 | 0,5 | | |
| | Tapioca Stärke | | 0,5 | | | |
| B | Titanium dioxide, gecoated | 1,0 | 0,5 | 3,0 | 2,0 | 4,0 |
| | Zinc oxide | 5,0 | 10,0 | 2,0 | 3,0 | |
| C | Ammonium Acryloyldimethyltaurate/ Beheneth-25 Methacrylate Crosspolymer | 0,2 | | 1,0 | 0,5 | |
| D | Aqua | ad 100 | ad 100 | ad 100 | ad 100 | ad 100 |
| | Hydroxypropyl Methylcellulose | | | 0,1 | | 0,05 |
| | Sorbitol | 5,0 | 7,0 | 8,5 | 3,0 | 4,5 |
| | PUR-Verdicker A.1 | 3,0 | 5,0 | 0,9 | 1,4 | 2,0 |
| E | Mixed parabens | 0,3 | 0,6 | 0,2 | | 0,4 |
| | Phenoxyethanol | 0,2 | 0,3 | 0,4 | 0,5 | 0,4 |
| | Diazolidinyl Harnstoff | | | 0,23 | 0,2 | |
| F | Parfum | 0,2 | | 0,3 | 0,1 | |

### Herstellung:

Phase A auf 80°C erwärmen.

Phase B in Phase A geben und 3 min homogenisieren. Phase C einrühren.

Cellulose (wenn benötigt) in Wasser vorquellen lassen, dann die restlichen Inhaltsstoffe der Phase D zugeben und auf 80°C erwärmen.

Phase D in Phase A+B+C einrühren und homogenisieren. Emulsion unter Rühren auf ca. 40°C abkühlen und Phase E und F zugeben. Unter Rühren auf RT abkühlen und homogenisieren.

Anstelle der feststoffstabilisierten Emulsion enthaltend Polyurethan-Verdicker A.1 werden auch feststoffstabilisierte Emulsionen enthaltend eines oder mehrere der Polyurethane A.2, A.3, A.4 oder A.5 hergestellt.

### Sonnenschutzcreme

| Phase | Inhaltsstoff /INCI | F.6.1 | F.6.2 | F.6.3 | F.6.4 | F.6.5 |
|---|---|---|---|---|---|---|
| A | Aqua | ad 100 | ad 100 | ad 100 | ad 100 | ad 100 |
| | Disodium EDTA | 0,1 | 0,1 | 0,1 | 0,1 | 0,1 |
| | Glycerin | 3,00 | 7,50 | 8,0 | 7,50 | 5,00 |
| | Benzophenone-4 | 2,0 | | | 4,0 | |
| | Phenylbenzimidazol Sulfonsäure | | 0,50 | 4,00 | | 8,0 |
| | Triethanolamine | 1,0 | 0,25 | 2,0 | 2,0 | 4,0 |
| | Panthenol | 0,5 | | 0,75 | | 1,0 |
| | PUR-Verdicker A.1 | 2,5 g | 0,5 g | 2,0 g | 4,0 | 1,5 |
| | Xanthan gum | | 0,3 | 0,15 | | 0,2 |
| B | Octocrylene | 8,0 | | | 7,5 | |
| | Ethylhexyl Methoxycinnamate, Diethylamino Hydroxybenzoyl Hexyl Benzoate | 5,0 | 10,0 | 8,0 | 3,0 | 7,0 |
| | Steareth-21 | 2,0 | 3,0 | | 2,5 | |
| | Steareth-2 | 1,5 | | | | |
| | PEG-40 Stearat | | | 1,0 | | 2,0 |
| | Glycerinmonostearat SE | | 1,0 | 3,0 | 1,5 | 1,5 |
| | Dibutyl Adipate | 3,0 | 5,0 | 3,5 | 2,5 | 2,0 |
| | Cetearyl Alcohol | 2,0 | | | 0,5 | 3,0 |
| | Stearyl Alcohol | 1,5 | 3,0 | 2,5 | 0,6 | 2,0 |
| | Butyrospermum Parkii (Shea Butter) | 1,0 | 0,5 | | 1,0 | 1,5 |
| | Dimethicone | 1,0 | 0,5 | 1,5 | 0,8 | 2,0 |
| | PVP Hexadecene Copolymer | 0,20 | | 0,50 | 0,8 | 1,00 |
| | Bisabolol | 0,2 | 0,1 | | | 0,3 |
| C | DMDM Hydantoin | 0,5 | 0,5 | 0,5 | 0,5 | 0,75 |
| | Water, Aloe Barbadensis Leaf Juice | | | 0,5 | 1,0 | |
| | Alpha-Glucosilrutin | 0,60 | 0,5 | 0,4 | 0,25 | 0,3 |
| | Parfum | 0,10 | 0,25 | 0,30 | 0,40 | 0,20 |

### Herstellung:

Die Phasen A und B getrennt auf ca. 80°C erwärmen.

Phase A in Phase B einrühren und kurz homogenisieren.

Unter Rühren auf ca. 40°C abkühlen. Komponenten der Phase C nacheinander zur Emulsion geben und unter Rühren auf Raumtemperatur abkühlen. Kurz homogenisieren.

Anstelle der Sonnenschutzcreme enthaltend Polyurethan-Verdicker A.1 werden auch Sonnenschutzcremes enthaltend eines oder mehrere der Polyurethane A.2, A.3, A.4 oder A.5 hergestellt.

### Gesichtscreme mit Sodium Ascorbyl Phosphate

| Phase | Inhaltsstoff/ INCI | F.7.1 | F.7.2 | F.7.3 | F.7.4 | F.7.5 |
|---|---|---|---|---|---|---|
| A | Water | ad 100 | ad 100 | ad 100 | ad 100 | ad 100 |
| | Butylene Glycol | 5,0 | 6,5 | 5,5 | 3,5 | 4,0 |
| | PUR-Verdicker A.1 | 3,5 | 1,5 | 2,5 | 5,0 | 2,0 |
| | Xanthan Gum | 0,25 | | 0,2 | 0,1 | |
| | Potasium Sorbate | 0,1 | 0,1 | 0,1 | 0,1 | 0,1 |
| | Imidazolidinyl Urea | 0,3 | | 0,2 | | |
| B | Potassium Cetyl Phosphate | 1,5 | 2,0 | 1,9 | 2,5 | 1,0 |
| | Caprylic/Capric Triglyceride | 2,0 | 5,0 | 3,0 | 4,0 | 2,5 |
| | Stearyl Alcohol | 0,5 | 1,5 | 2,0 | 1,0 | 3,0 |
| | Cetearyl Alcohol, Dicetyl Phosphate, Ceteth-10 Phosphate | 1,5 | 2,0 | 1,8 | 1,9 | 2,1 |
| | Simmondsia Chinensis (Jojoba) Seed Oil | 3,0 | 1,5 | 0,5 | 1,0 | 2,5 |
| | Mineral Oil | 2,0 | 5,0 | 10,0 | 7,5 | 4,0 |
| | Parabenmischung | 0,2 | 0,2 | 0,2 | 0,2 | 0,2 |
| C | Sodium Ascorbyl Phosphate | 5,0 | 2,0 | 3,0 | 4,0 | 1,5 |
| | Lactic acid | q.s. | q.s. | q.s. | q.s. | q.s. |
| | Water | 10,0 | 4,0 | 5,0 | 10,0 | 3,0 |
| D | Tocopherol | 0,1 | | | 0,2 | |
| | Retinol | | 0.03 | 0,025 | | 0,05 |
| E | Fragrance | 0,1 | 0,05 | 0,05 | 0,1 | 0,15 |

### Herstellung

Die Phasen A und B getrennt auf ca. 80°C erwärmen.

Phase A in Phase B einrühren und homogenisieren.

Phase C in Phase A+B einrühren und homogenisieren.
Unter Rühren auf ca. 40°C abkühlen. PH-Wert der Phase C mit Milchsäure auf <6,5 einstellen. Phase C zugeben und unter Rühren auf ca. 30°C abkühlen. Phase D und E zugeben. Unter Rühren auf Raumtemperatur abkühlen und kurz homogenisieren.

Hinweis: pH-Wert der Emulsion < 6,5 mit Milchsäure einstellen

Anstelle der Gesichtscreme enthaltend Polyurethan-Verdicker A.1 werden auch Gesichtscremes enthaltend eines oder mehrere der Polyurethane A.2, A.3, A.4 oder A.5 hergestellt.

### Hydroxycarbonsäure-Creme

| Phase | Inhaltsstoff/ INCI | F.8.1 | F.8.2 | F.8.3 |
|---|---|---|---|---|
| A | Ceteareth-6, Stearyl Alcohol | 2,0 | | 2,5 |
| | Ceteareth-25 | 2,0 | | 2,5 |
| | PEG-100 Stearate, Glyceryl Stearate | | 3,5 | 0,5 |
| | Polyglyceryl-3 Distearate | | 2,0 | |
| | Mineral Oil | 8,0 | 3,5 | 5,0 |
| | Cetearyl Ethylhexanoate | 7,0 | 5,5 | 4,0 |
| | Sorbitan Stearate | 0,5 | 1,5 | 0,5 |
| | Cera Alba | | 0,5 | 1,0 |
| | Cetyl Alcohol | 1,5 | 3,5 | 4,0 |
| | Dimethicone | 0,2 | 2,0 | 0,5 |
| B | Panthenol | 1,0 | 0,5 | 0,3 |
| | Propylene Glycol | 3,0 | 2,0 | 5,0 |
| | PUR-Verdicker A.1 | 1,0 | 3,0 | 5,0 |
| | Hydroxysäure | 3,0 | 7,0 | 10,0 |
| | Aqua | ad 100 | ad 100 | ad 100 |
| C | Sodium Hydroxide | q.s. | q.s. | q.s. |
| D | Bisabolol | 0,2 | 0,1 | 0,3 |
| | preservative | q.s. | q.s. | q.s. |
| | Fragrance | q.s. | q.s. | q.s. |

### Hinweis

Alpha-Hydroxysäuren: Milchsäure, Citronensäure, Äpfelsäure, Glycolsäure

Di-Hydroxysäure: Weinsäure

Beta-Hydroxysäure: Salicylsäure

pH -Wert > 3 einstellen

### Herstellung

Phase A und B getrennt auf ca. 80°C erwärmen. pH-Wert der Phase B gegebenenfalls mit Na-OH auf >3 einstellen. Phase B in Phase A einrühren, kurz homogenisieren.

Abkühlen auf ca. 40°C unter Rühren, Komponenten der Phase D nacheinander zugeben, nochmals homogenisieren.

Anstelle der Hydroxycarbonsäurecreme enthaltend Polyurethan-Verdicker A.1 werden auch Hydroxycarbonsäurecremes enthaltend eines oder mehrere der Polyurethane A.2, A.3, A.4 oder A.5 hergestellt.

### Emulsion mit Deo-Wirkstoff

| Phase | Inhaltsstoff/ INCI | F.9.1 | F.9.2 | F.9.3 | F.9.4 | F.9.5 |
|---|---|---|---|---|---|---|
| | Ceteareth-6, Stearyl Alcohol | 1,5 | 2,0 | | | 1,0 |
| | Ceteareth-25 | 1,5 | 0,5 | | | 1,0 |
| | PEG-40 Hydrogenated Castor Oil | 0,5 | 1,0 | 2,0 | | |
| | Glyceryl Stearate | | 0,5 | 2,0 | 1,0 | |
| | Cetyl Alcohol | 2,0 | 1,0 | 0,5 | 2,5 | 0,2 |
| | Hydrogenated CocoGlycerides | 2,0 | | | 1,0 | 0,5 |
| | Hydrogenated Pölyisobutene | | 10,0 | 20,0 | 5,0 | 3,0 |
| | Decyl Oleate | 3,0 | 2,0 | | 8,0 | 5,0 |
| | Bis-PEG/PPG-14/14 Dimethicone, Cyclopentasiloxane | 3,0 | 3,5 | 4,0 | 2,0 | 1,5 |
| | Talc | 3,0 | 2,5 | | | 1,5 |
| | Magnesium Aluminum Silicate | 1,0 | | 0,5 | 1,0 | 1,5 |
| B | Propylene Glycol | 10,0 | 5,0 | 7,5 | 20,0 | 15,0 |
| | PUR-Verdicker A.1 | 0,5 | 1,0 | 3,0 | 3,5 | 2,0 |
| | Xanthan gum | 0,2 | 0,1 | | | 0,05 |
| | Cetyl Hydroxyethylcellulose | 0,3 | | | 0,1 | |
| | Aluminum Chlorohydrate | 5,0 | 10,0 | 20,0 | | |
| | Aluminum Zirconium Tetrachlorohydrex GLY | | 15,0 | | 50,0 | 20,0 |
| | Aqua | ad 100 | ad 100 | ad 100 | ad 100 | ad 100 |
| C | Neutralisationsmittel | q.s. | q.s. | q.s. | q.s. | q.s. |
| D | Alcohol | 5,0 | 10,0 | 25,0 | 7,5 | 6,0 |
| | Allantoin | 0,1 | 0,1 | 0,1 | 0,1 | 0,1 |
| | preservative | q.s. | q.s. | q.s. | q.s. | q.s. |
| | Fragränce | q.s. | q.s. | q.s. | q.s. | q.s. |

### Herstellung

Die Phasen A und B getrennt auf ca. 80°C erwärmen.

Phase B unter Homogenisieren in Phase A einrühren. Mit Phase C gegebenenfalls auf pH 4-5 einstellen. Abkühlen auf ca. 40°C, die Phase D zugeben und unter Rühren auf Raumtemperatur abkühlen lassen. Kurz homogenisieren.

### Hinweis: pH-Wert der Emulsion auf 4-5 einstellen

Anstelle der Emulsion mit Deo-Wirkstoff enthaltend Polyurethan-Verdicker A.1 werden auch Emulsionen mit Deo-Wirkstoff enthaltend eines oder mehrere der Polyurethane A.2, A.3, A.4 oder A.5 hergestellt.

### Enthaarungscreme

| Phase | Inhaltsstoff/ INCI | F.10.1 | F.10.2 | F.10.3 |
|---|---|---|---|---|
| A | Glyceryl Stearate | 1,0 | | |
| | Ceteareth-12 | | 1,0 | 2,0 |
| | Ceteareth-20 | | 1,0 | 2,0 |
| | Stearyl Alcohol | | 4,0 | 1,0 |
| | Cetyl Alcohol | 4,0 | | 1,0 |
| | Mineral Oil | | 6,0 | 4,0 |
| | Prunus Armeniaca (Apricot) Kernel Oil | 3,0 | 1,0 | 2,0 |
| B | Propylene Glycol | 1,0 | 2,0 | 10,0 |
| | Calcium Carbonate | 10,0 | | |
| | Calcium Hydroxide | 7,0 | | |
| | Sodium Hydroxide | | 0,4 | 0,6 |
| | Calcium Thioglycolate | 5,0 | 3,0 | 5,0 |
| | PUR-Verdicker A.1 | 3,0 | 1,5 | 2,0 |
| | Aqua | ad 100 | ad 100 | ad 100 |
| C | Tocopherol | 0,1 | 0,2 | 0,15 |
| | Bisabolol | 0,2 | 0,1 | 0,3 |
| | Fragrance | q.s. | q.s. | q.s. |

### Herstellung

Phase A und B getrennt auf ca. 80°C erwärmen.

Phase B in Phase A unter Homogenisieren einrühren, kurz homogenisieren.

Abkühlen auf ca. 40°C, Phase C zugeben, unter Rühren auf RT abkühlen und nochmals homogenisieren.

Hinweis: pH-Wert der Emulsion auf > 10 einstellen

Anstelle der Enthaarungscreme enthaltend Polyurethan-Verdicker A.1 werden auch Enthaarungscremes enthaltend eines oder mehrere der Polyurethane A.2, A.3, A.4 und A.5 hergestellt.

### Selbstbräunungsemulsion

| Phase | Inhaltsstoff/ INCI | F.11.1 | F.11.2 | F.11.3 | F.11.4 |
|---|---|---|---|---|---|
| A | Isohexadecane | 4,0 | 2,0 | 3,0 | 1,0 |
| | Dimethicone | 1,0 | 1,0 | 0,5 | 1,5 |
| | Cetearyl Alcohol | 2,0 | 2,5 | 1,5 | 2,5 |
| | Isopropyl Myristate | 1,0 | | 2,0 | 3,0 |
| | Simmondsia Chinensis (Jojoba) Seed Oil | 2,0 | 1,0 | 0,5 | 0,5 |
| | Polyglyceryl-3 Methylglucose Distearate | 3,0 | | 3,5 | |
| | PEG-40 Stearate | | 2,5 | | 2,0 |
| | Lecithin | 0,5 | | | 1,0 |
| | Cetearyl Glucoside | 0,5 | | 0,5 | |
| | Sorbitan Oleate | | 0,5 | 0,5 | 0,3 |
| B | Glycerin | 4,0 | | | 5,0 |
| | Butylene Glycol | | 4,0 | 3,0 | |
| | PUR-Verdicker A.1 | 1,0 | 3,0 | 1,5 | 2,5 |
| | Xanthan Gum | 0,1 | | | 0,1 |
| | Aqua | ad 100 | ad 100 | ad 100 | ad 100 |
| C | Dihydroxyacetone | 1,5 | 5,0 | | |
| | Erythrulose | | | 2,0 | 4,0 |
| | Aqua | 5,0 | 10,0 | 5,0 | 8,0 |
| | Citric Acid | q.s. | q.s. | q.s. | q.s. |
| D | Bisabolol | 0,3 | 0,5 | 0,2 | 0,4 |
| | Tocopheryl Acetate | 0,7 | 0,5 | 0,6 | 1,0 |
| | Preservative | q.s. | q.s. | q.s. | q.s. |
| | Fragrance | q.s. | q.s. | q.s. | q.s. |

### Herstellung

Die Phasen A und B getrennt auf ca. 80°C erwärmen.

Phase B in Phase A einrühren und kurz homogenisieren.

Unter Rühren auf ca. 40°C abkühlen, Phase C hinzugeben und unter Rühren auf ca. 30°C abkühlen. Komponenten der Phase D nacheinander zugeben und unter Rühren auf RT abkühlen. Kurz homogenisieren.

Hinweis: pH-Wert der Emulsion auf 4-5,5 einstellen

Anstelle der Selbstbräunungsemulsion enthaltend Polyurethan-Verdicker A.1 werden auch Selbstbräunungsemulsionen enthaltend eines oder mehrere der Polyurethane A.2, A.3, A.4 oder A.5 hergestellt.

### Conditioner Shampoo

| Inhaltsstoff/ INCI | F.12.1 | F.12.2 | F.12.3 | F.12.4 |
|---|---|---|---|---|
| Aqua | ad 100 | ad 100 | ad 100 | ad 100 |
| Sodium Laureth Sulfate | 35,7 | | 30,0 | 12,0 |
| Cocamidopropyl Betaine | 13,5 | 15,0 | | |
| Disodium Cocoamphodiacetate | | 10,0 | | |
| Sodium Cocoamphoacetate | | | 6,0 | |
| Polysorbate 20 | | 5,0 | | |
| Decyl Glucoside | | 5,0 | | 1,5 |
| Laureth-3 | | 2,0 | | |
| Sodium Laureth Sulfate, Glycol Distearate, Cocamide MEA, Laureth-10 | | | 3,0 | 2,0 |
| Coco-Glucoside, Glyceryl Oleate | | | | 5,0 |
| Dimethicone | | | 2,0 | |
| Conditioning polymer | 2,0 | 0,5 | 0,75 | 0,4 |
| PUR-Verdicker A.1 | 0,75 | 1,2 | 0,5 | 1,0 |
| PEG-150 Distearate | | 3,0 | | |
| Citric Acid | | q.s. | q.s. | |
| Preservative | q.s. | q.s. | q.s. | q.s. |
| Fragrance | q.s. | q.s. | q.s. | q.s. |
| Dye | q.s. | q.s. | q.s. | q.s. |
| Sodium Chloride | | | 1,0 | 1,0 |

Unter Conditioningpolymer wird verstanden Polyquaternium-7, PQ-10, PQ-16, PQ-39, PQ-44, PQ-46, PQ-67, Guarhydroxypropyltrimonium Chloride, PQ-87 sowie Kombinationen aus diesen.

Anstelle des Conditioner Shampoos enthaltend Polyurethan-Verdicker A.1 werden auch Conditioner Shampoos enthaltend eines oder mehrere der Polyurethane A.2, A.3, A.4 und A.5 hergestellt.

### Hair Conditioner

| Phase | Inhaltsstoff/ INCI | F.13.1 | F.13.2 | F.13.3 | F.13.4 | F.13.5 |
|---|---|---|---|---|---|---|
| A | Water | ad 100 | ad 100 | ad 100 | ad 100 | ad 100 |
| | PUR-VerdickerA.1 | 2,5 | 1,5 | 3,0 | 0,6 | 2,0 |
| | Hydroxyethylcellulose | 0,05 | 0,1 | | 0,2 | |
| | Propylene Glycol | 1,0 | 2,0 | | 0,8 | 0,5 |
| | Panthenol | 0,5 | | 0,75 | 0,25 | 0,3 |
| B | Quaternium-91, Cetearyl Alcohol, Cetrimonium Methosulfate | 2,0 | | | | 1,5 |
| | Distearoyethyl Hydroxyethylmonium Methosulfate, Cetearyl Alcohol | | | 3,0 | 4,0 | |
| | Hydrogenated Polyisobutene | 1,0 | 1,5 | | 1,0 | |
| | Cyclopentasiloxane | | | 2,0 | 1,0 | 0,5 |
| | Isopropylpalmitate | | 1,0 | | 2,0 | |
| | Persea Gratissima (Avocado) Oil | | | | | 2,5 |
| | Steareth-2 | 0,75 | | 0,5 | | |
| | Ceteareth-6, Stearyl Alcohol | | 1,5 | | | 0,5 |
| | Ceteareth-25 | | 1,5 | | | |
| | Cetearyl Alcohol | 2,0 | 1,5 | 0,5 | 4,0 | |
| C | Acrylat/C10-30 Alkylacrylat-Copolymer | 0,1 | | | 0,2 | 0,15 |
| D | Cetrimonium Chloride | 1,5 | | 3,0 | | |
| | Conditioning-Polymer | 2,0 | 6,0 | 3,0 | 1,5 | 0,8 |
| E | Preservative | q.s. | q.s. | q.s. | q.s. | q.s. |
| | Fragrance | q.s. | q.s. | q.s. | q.s. | q.s. |

Unter Conditioningpolymer wird verstanden Polyquaternium-7, PQ-10, PQ-16, PQ-39, PQ-44, PQ-46, PQ-67, Guarhydroxypropyltrimonium Chloride, PQ-87 sowie Kombinationen aus diesen.

### Herstellung

Die Phasen A und B getrennt auf ca. 80°C erwärmen.

Phase C in Phase B einrühren, anschließend Phase A in Phase B/C einrühren und kurz homogenisieren.

Unter Rühren auf ca. 50°C abkühlen, Komponenten der Phase D nacheinander hinzugeben und unter Rühren auf ca. 30°C abkühlen. Komponenten der Phase E nacheinander zugeben und unter Rühren auf RT abkühlen. Kurz homogenisieren.

Anstelle des Hair Conditioners enthaltend Polyurethan-Verdicker A.1 werden auch Hair Conditioner enthaltend eines oder mehrere der Polyurethane A.2, A.3, A.4 oder A.5 hergestellt.

## Patentansprüche

1. Verfahren zur Herstellung von Polyurethanen umfassend
I) mindestens zwei hydrophile Abschnitte S,
II) mindestens einen von S verschiedenen hydrophilen Abschnitt P,
III) mindestens zwei endständige hydrophobe Abschnitte T,
IV) mindestens zwei von T verschiedene hydrophobe Abschnitte D,
wobei
a) sich an jeden Abschnitt T ein Abschnitt S unmittelbar anschließt,
b) sich an jeden Abschnitt S auf mindestens einer Seite mindestens ein Abschnitt D anschließt,
c) sich an jeden Abschnitt P mindestens zwei Abschnitte D anschließen,
**dadurch gekennzeichnet,**
**dass** die Herstellung in Gegenwart von mindestens einem Carbonsäuresalz mindestens eines Metalls ausgewählt aus der Gruppe bestehend aus den Alkalimetallen, den Erdalkalimetallen und deren Mischungen erfolgt.

2. Verfahren nach Anspruch 1, wobei wenigstens ein Teil der Polyurethane Allophanat-Segmente enthält.

3. Verfahren nach mindestens einem der Ansprüche 1 oder 2, wobei wenigstens ein Teil der Polyurethane Isocyanurat-Segmente enthält.

4. Verfahren nach mindestens einem der Ansprüche 1 bis 3, **dadurch gekennzeichnet, dass** die auf die Reaktionsmischung bezogene Menge an Lösungsmitteln, die unterschiedlich sind von den Substanzen, die die hydrophilen und hydrophoben Abschnitte in die Polyurethane einbringen, im Bereich von 0 bis 10 Gew.-% beträgt.

5. Verfahren nach mindestens einem der Ansprüche 1 bis 4, **dadurch gekennzeichnet, dass** das Verfahren einstufig ist.

6. Verfahren nach mindestens einem der Ansprüche 1 bis 5, **dadurch gekennzeichnet, dass** zusätzlich auch Polyurethane erhalten werden, die
I) mindestens zwei hydrophile Abschnitte S,
II) keinen hydrophilen Abschnitt P,
III) mindestens zwei endständige hydrophobe Abschnitte T,
IV) mindestens einen von T verschiedenen hydrophoben Abschnitt D umfassen,
wobei
a) sich an jeden Abschnitt T ein Abschnitt S unmittelbar anschließt,
b) sich an jeden Abschnitt S ein Abschnitt D anschließt.

7. Verfahren nach mindestens einem der Ansprüche 1 bis 6, **dadurch gekennzeichnet, dass** der mindestens eine hydrophile Abschnitt P ein zahlenmittleres Molekulargewicht von 4000 bis 12000 g/mol aufweist.

8. Verfahren nach mindestens einem der Ansprüche 1 bis 7, **dadurch gekennzeichnet, dass** die Herstellung in Gegenwart von mindestens einem Kalium-Carboxylat erfolgt.

9. Verfahren nach mindestens einem der Ansprüche 1 bis 8, **dadurch gekennzeichnet, dass** die Herstellung in Gegenwart von weniger als 10 ppm Zinn erfolgt.

10. Verfahren nach mindestens einem der Ansprüche 1 bis 9, **dadurch gekennzeichnet, dass** die Herstellung in Gegenwart von weniger als 10 ppm Zink erfolgt.

11. Verfahren nach mindestens einem der Ansprüche 1 bis 10, **dadurch gekennzeichnet, dass** zur Herstellung der Polyurethane jeweils mindestens
- ein alkoxylierter C₄-C₃₀-Alkohol,
- ein Polyetherdiol und
- ein Diisocyanat
eingesetzt werden.

12. Verfahren nach Anspruch 11, **dadurch gekennzeichnet, dass**
- wenigstens ein C₄-C₃₀-Alkohol ein verzweigtes C₁₂-C₃₀-Alkanol ist,
- wenigstens ein Polyetherdiol ein Molekulargewicht Mₙ im Bereich von 4000 bis 12000 g/mol aufweist und
- wenigstens ein Diisocyanat ein aliphatisches Diisocyanat ist.

13. Verwendung von Polyurethanen erhältlich nach einem Verfahren nach den Ansprüchen 1 bis 12 in wässrigen Zubereitungen.

14. Zubereitungen enthaltend mindestens ein Polyurethan erhältlich nach einem Verfahren nach den Ansprüchen 1 bis 12.

## Claims

1. A process for preparing polyurethanes comprising
I) at least two hydrophilic sections S,
II) at least one hydrophilic section P different from S,
III) at least two terminal hydrophobic sections T,
IV) at least two hydrophobic sections D different from T,
where
a) to each section T is directly attached a section S,
b) to each section S on at least one side is attached at least one section D,
c) to each section P are attached at least two sections D,
wherein the preparation takes place in the presence of at least one carboxylic acid salt of at least one metal selected from the group consisting of the alkali metals, the alkaline earth metals and mixtures thereof.

2. The process according to claim 1, where at least some of the polyurethanes comprise allophanate segments.

3. The process according to at least one of claims 1 and 2, where at least some of the polyurethanes comprise isocyanurate segments.

4. The process according to at least one of claims 1 to 3, wherein the amount of solvents which are different from the substances which introduce the hydrophilic and hydrophobic sections into the polyurethanes, based on the reaction mixture, is in the range from 0 to 10% by weight.

5. The process according to at least one of claims 1 to 4, wherein the process is single-stage.

6. The process according to at least one of claims 1 to 5, wherein polyurethanes are also additionally obtained which comprise
I) at least two hydrophilic sections S,
II) no hydrophilic section P,
III) at least two terminal hydrophobic sections T,
IV) at least one hydrophobic section D different from T,
where
a) to each section T is directly attached a section S,
b) to each section S is attached a section D.

7. The process according to at least one of claims 1 to 6, wherein the at least one hydrophilic section P has a number-average molecular weight of from 4000 to 12 000 g/mol.

8. The process according to at least one of claims 1 to 7, wherein the preparation takes place in the presence of at least one potassium carboxylate.

9. The process according to at least one of claims 1 to 8, wherein the preparation takes place in the presence of less than 10 ppm of tin.

10. The process according to at least one of claims 1 to 9, wherein the preparation takes place in the presence of less than 10 ppm of zinc.

11. The process according to at least one of claims 1 to 10, wherein in each case at least
- one alkoxylated C₄-C₃₀-alcohol,
- one polyetherdiol and
- one diisocyanate
are used for preparing the polyurethanes.

12. The process according to claim 11, wherein
- at least one C₄-C₃₀-alcohol is a branched C₁₂-C₃₀-alkanol,
- at least one polyetherdiol has a molecular weight Mₙ in the range from 4000 to 12 000 g/mol and
- at least one diisocyanate is an aliphatic diisocyanate.

13. The use of polyurethanes obtainable by a process according to claims 1 to 12 in aqueous preparations.

14. A preparation comprising at least one polyurethane obtainable by a process according to claims 1 to 12.

## Revendications

1. Procédé de fabrication de polyuréthanes, comprenant
I) au moins deux sections hydrophiles S,
II) au moins une section hydrophile P différente de S,
III) au moins deux sections hydrophobes terminales T,
IV) au moins deux sections hydrophobes D différentes de T,
a) une section S étant directement reliée à chaque section T,
b) au moins une section D étant reliée sur au moins un côté à chaque section S,
c) au moins deux sections D étant reliées à chaque section P,
**caractérisé en ce que**
la fabrication a lieu en présence d'au moins un sel d'acide carboxylique d'au moins un métal choisi dans le groupe constitué par les métaux alcalins, les métaux alcalino-terreux et leurs mélanges.

2. Procédé selon la revendication 1, dans lequel au moins une partie des polyuréthanes contiennent des segments allophanate.

3. Procédé selon au moins l'une quelconque des revendications 1 ou 2, dans lequel au moins une partie des polyuréthanes contiennent des segments isocyanurate.

4. Procédé selon au moins l'une quelconque des revendications 1 à 3, **caractérisé en ce que** la quantité de solvants différents des substances introduisant les sections hydrophiles et hydrophobes dans les polyuréthanes, relative au mélange réactionnel, est dans la plage allant de 0 à 10 % en poids.

5. Procédé selon au moins l'une quelconque des revendications 1 à 4, **caractérisé en ce que** le procédé est à une étape.

6. Procédé selon au moins l'une quelconque des revendications 1 à 5, **caractérisé en ce que** des polyuréthanes comprenant
I) au moins deux sections hydrophiles S,
II) aucune section hydrophile P,
III) au moins deux sections hydrophobes terminales T,
IV) au moins une section hydrophobe D différente de T,
sont également obtenus,
a) une section S étant directement reliée à chaque section T,
b) une section D étant reliée à chaque section S.

7. Procédé selon au moins l'une quelconque des revendications 1 à 6, **caractérisé en ce que** la ou les sections hydrophiles P présentent un poids moléculaire moyen en nombre de 4 000 à 12 000 g/mol.

8. Procédé selon au moins l'une quelconque des revendications 1 à 7, **caractérisé en ce que** la fabrication a lieu en présence d'au moins un carboxylate de potassium.

9. Procédé selon au moins l'une quelconque des revendications 1 à 8, **caractérisé en ce que** la fabrication a lieu en présence de moins de 10 ppm d'étain.

10. Procédé selon au moins l'une quelconque des revendications 1 à 9, **caractérisé en ce que** la fabrication a lieu en présence de moins de 10 ppm de zinc.

11. Procédé selon au moins l'une quelconque des revendications 1 à 10, **caractérisé en ce qu'**à chaque fois au moins
- un alcool en C₄-C₁₀ alcoxylé,
- un polyéther-diol et
- un diisocyanate
sont utilisés pour la fabrication des polyuréthanes.

12. Procédé selon la revendication 11, **caractérisé en ce que**
- au moins un alcool en C₄-C₃₀ est un alcanol en C₁₂-C₃₀ ramifié,
- au moins un polyéther-diol présente un poids moléculaire Mₙ dans la plage allant de 4 000 à 12 000 g/mol et
- au moins un diisocyanate est un diisocyanate aliphatique.

13. Utilisation de polyuréthanes pouvant être obtenus par un procédé selon les revendications 1 à 12 dans des préparations aqueuses.

14. Préparations contenant au moins un polyuréthane pouvant être obtenu par un procédé selon les revendications 1 à 12.
